(19) Europäisches Patentamt / European Patent Office / Office européen des brevets

(11) **EP 4 329 780 B1**

(12) **EUROPEAN PATENT SPECIFICATION**

(45) Date of publication and mention of the grant of the patent:
**26.11.2025 Bulletin 2025/48**

(21) Application number: **22727501.3**

(22) Date of filing: **29.04.2022**

(51) International Patent Classification (IPC):
*A61K 35/17* (2025.01)    *C07K 14/47* (2006.01)
*C07K 14/82* (2006.01)    *C07K 14/725* (2006.01)
*A61K 40/11* (2025.01)    *A61K 40/32* (2025.01)
*C12N 5/09* (2010.01)    *C12N 5/0783* (2010.01)
*G01N 33/569* (2006.01)    *G01N 33/68* (2006.01)
*G01N 33/574* (2006.01)    *A61K 40/42* (2025.01)
*A61P 35/00* (2006.01)

(52) Cooperative Patent Classification (CPC):
**C07K 14/4748; A61K 40/11; A61K 40/32; A61K 40/4201; A61P 35/00; C07K 14/7051; C12N 5/0636; C12N 5/0694; G01N 33/56977; G01N 33/574; G01N 33/6878;** A61K 2239/57; C12N 2510/00

(86) International application number:
**PCT/IL2022/050443**

(87) International publication number:
**WO 2022/229966 (03.11.2022 Gazette 2022/44)**

(54) **T CELL RECEPTORS DIRECTED AGAINST RAS-DERIVED RECURRENT NEOANTIGENS AND METHODS OF IDENTIFYING SAME**

T-ZELL-REZEPTOREN GEGEN RAS-ABGELEITETE WIEDERKEHRENDE NEOANTIGENE UND VERFAHREN ZUR IDENTIFIZIERUNG DAVON

RÉCEPTEURS DES LYMPHOCYTES T DIRIGÉS CONTRE DES NÉOANTIGÈNES RÉCURRENTS DÉRIVÉS DE RAS ET LEURS PROCÉDÉS D'IDENTIFICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.04.2021  IL 28281421**
**19.07.2021  US 202163223114 P**

(43) Date of publication of application:
**06.03.2024 Bulletin 2024/10**

(73) Proprietor: **Yeda Research and Development Co. Ltd**
**7610002 Rehovot (IL)**

(72) Inventors:
• **PERI, Aviyah**
**7610002 Rehovot (IL)**
• **GREENSTEIN, Erez**
**7610002 Rehovot (IL)**

• **ALON, Michal**
**7610002 Rehovot (IL)**

(74) Representative: **Boult Wade Tennant LLP**
**Salisbury Square House**
**8 Salisbury Square**
**London EC4Y 8AP (GB)**

(56) References cited:
WO-A2-2020/234875

• HONDOWICZ BRIAN D. ET AL: "Discovery of T Cell Antigens by High-Throughput Screening of Synthetic Minigene Libraries", PLOS ONE, vol. 7, no. 1, 12 January 2012 (2012-01-12), pages e29949, XP055937170, Retrieved from the Internet <URL:https://journals.plos.org/plosone/article/file?id=10.1371/journal.pone.0029949&type=printable> DOI: 10.1371/journal.pone.0029949

Remarks:
    The complete document including Reference Table(s) and the Sequence Listing(s) can be downloaded from the EPO website

**Description**

RELATED APPLICATIONS

This application claims the benefit of priority of U.S. Provisional Patent Application, 63/223,114 filed 19 July 2021 and Israeli Patent Application 282814 filed 29 April 2021.

SEQUENCE LISTING STATEMENT

The ASCII file, entitled 91651SequenceListing.txt, created on 26 April, 2022, comprising 9,217 bytes, is submitted concurrently with the filing of this application

FIELD AND BACKGROUND OF THE INVENTION

The present invention, in some embodiments thereof, relates to identification of T cell receptors which bind to recurrent mutated neopeptides, and more specifically to Ras-derived mutated neopeptides.
Immunotherapy sparked new hope for oncology in recent years, due to its remarkable ability to induce long-term tumor regression of metastatic cancer. This feature is shared across immunotherapeutic modalities, including both checkpoint blockade and adoptive cell transfer (ACT) of TILs. It is believed that the final common pathway of these two treatments is specific recognition of tumor antigens by cytotoxic T-lymphocytes. Specifically, with the advancement of sequencing capabilities, the in-depth dissection of immunotherapy success stories has revealed a center-stage role for mutation-derived antigens, designated *neo-antigens,* in mediating an anti-tumor immune response.
Neo-antigens are cell-surface peptide/human-leukocyte antigen (HLA) complexes where the peptide component, i.e., the neo-peptide, is the altered degradation product of a mutated protein. Restricted in expression to the diseased tissue, and uncurbed by immune tolerance, neo-antigens may elicit specific anti-tumor reactivity upon TCR engagement, and are therefore ideal therapeutic targets.
The great majority of neo-antigens identified from treated patients derive from private, non-recurring, mutations, and thus, although effective, cannot be generalized beyond the individual patient. Hotspot neo-antigens, i.e., neo-antigens that appear in a large group of cancer patients, clearly form only at the intersection of recurrent oncogenic mutations and common HLA alleles. Such neo-antigens are highly sought after for two main reasons. First, hotspot neo-antigens may pave the way toward "off-the-shelf" cellular treatments, vaccines and patient screening strategies. Tumor cells expressing validated mutation/HLA combinations should be amenable to immunotherapy. Even in the absence of *a priori* immune recognition, pre-determined TCRs, from other patients or even healthy donors, can be used to redirect autologous T-cells against neglected hotspot neo-antigens. Moreover, neo-antigen-specific T-cells, undetectable prior to treatment, have been shown to expand significantly following mutation-based vaccines. Second, hotspot neo-antigens are potentially superior to private neo-antigens as treatment targets. This is because immunotherapy directed at sub-clonal mutations of heterogeneous tumors might give way to immune escape, whereas hotspot neo-antigens, which are derived from clonal oncogenic mutations, are expected to present more homogenously within tumors.
The several hotspot neo-antigens uncovered over the years stem from major oncogenes, such as BRAF, NRAS and p53. The clinical relevance of such neo-antigens, however, was directly demonstrated through successful ACT treatment of a patient with metastatic colon cancer, utilizing autologous TILs directed at a newly discovered HLA-C*08:02/KRAS.G12D hotspot neo-antigen. These recent successes propelled the reemergence of endeavors to discover hotspot neo-antigens. A p53-centered screen revealed native TIL reactivity toward derived neo-antigens in 8% of screened patients, with hotspot neo-antigens presenting in several cases. Other efforts focused on the identification of T-cells from the peripheral blood of patients or healthy donors that target specific hotspots, thereby expanding the repertoire of known KRAS and other oncogene-derived neo-antigens for both HLA class-I and HLA class-II.
To date, neo-antigen discovery efforts are almost exclusively T-cell centric. In these methods, candidate neo-peptides are artificially expressed in antigen presenting cells (APCs), either as pulsed synthetic peptides or via minigene overexpression. APCs are then co-incubated with T-cells, most commonly TILs, and their response profile interpreted for indirect identification of neo-antigens. Further characterization and validation rely heavily on *in silico* binding predictions, such that identified neo-antigens are restricted to those that are both predicted to bind and are immunogenic in the tested patient. Moreover, irrelevant neo-antigens, which were edited out from the presented repertoire in tumor evolution, will nonetheless be identified as long as they were once immunogenic.
The causal role of RAS proteins in cancer has long been recognized, with activating mutations appearing in a third of all human cancers. The three main isoforms, KRAS, NRAS and HRAS, share an identical, 86-amino-acid-long N-termini. Within this identical stretch, three mutational hotspots were recognized: at positions 12, 13 and 61. Pan-cancer, KRAS is the most highly mutated RAS isoform (85% of RAS mutations). However, in melanoma, the most successful immunotherapy target to date, NRAS mutations dominate. Specifically, NRAS.61 is the second most highly mutated position in melanoma, appearing in as many as 20% of patients. NRAS-mutant melanoma is associated with poorer outcomes, compared with non-NRAS-mutant melanoma. The multiple attempts to develop RAS-targeted therapy have yet to yield effective, specifically approved therapies for NRAS-mutant melanoma.

Background art includes WO2020/234875, WO/2020/037302, WO/2019/075112; WO/2020/180648; WO/2019/226939; WO/2019/168984; WO/2019/226941 WO/2019/133853; WO/2019/036688; WO/2018/227030 WO/2019/050994; WO/2018/208856; WO/2018/195357; WO/2018/098362; WO/2019/104203; and WO/2017/106638.

SUMMARY OF THE INVENTION

According to an aspect of the present invention there is provided population of T cells for use in treating cancer, wherein an alpha chain of a TCR of at least 10 % of the T cells of the population has a CDR3 amino acid sequence as set forth in SEQ ID NO: 6, and a beta chain of the CDR has a CDR3 amino acid sequence as set forth in SEQ ID NO: 7.

According to embodiments of the present invention, the subject is HLA-A*01:01/NRAS.Q61K or HLA-A*01:01/NRAS.Q61R,

According to an aspect of the present invention there is provided an isolated population of T cells genetically modified to express a T cell receptor (TCR), wherein an alpha chain of the TCR has a CDR3 amino acid sequence as set forth in SEQ ID NO: 6, and a beta chain of the TCR has a CDR3 amino acid sequence as set forth in SEQ ID NO: 7.

According to an aspect of the present invention there is provided an isolated population of T cells, wherein an alpha chain of a TCR of at least 10 % of the T cells of the population has a CDR3 amino acid sequence as set forth in SEQ ID NO: 6, and a beta chain of the TCR has a CDR3 amino acid sequence as set forth in SEQ ID NO: 7.

According to embodiments of the present invention, T cells are autologous to the subject.

According to embodiments of the present invention, T cells are non-autologous to the subject.

According to embodiments of the present invention, T cells are genetically modified to express the T cell receptor.

According to embodiments of the present invention, T cells comprise CD8+ T cells.

According to embodiments of the present invention, cancer is selected from the group consisting of melanoma, colon cancer, breast cancer, thyroid cancer, stomach cancer, colorectal cancer, leukemia cancer, bladder cancer, lung cancer, ovarian cancer, breast cancer and prostate cancer.

According to embodiments of the present invention, cancer is melanoma.

According to embodiments of the present invention, the subject is being treated with a checkpoint inhibitor.

According to embodiments of the present invention, the isolated population of T cells are genetically modified to express the TCR.

According to embodiments of the present invention, the T cells are CD8+ T cells.

According to embodiments of the present invention, the isolated population of T cells is for the treatment of cancer.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

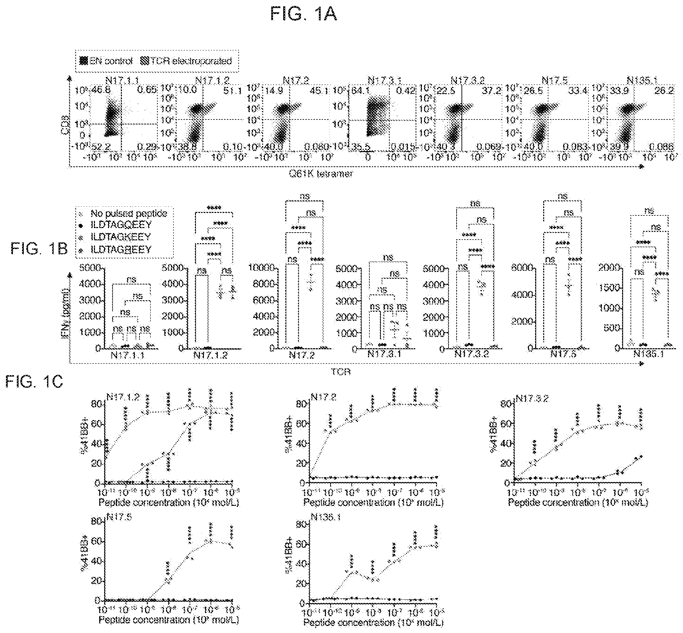

FIGs. 1A-C: A repertoire of potent neoantigen-specific TCRs. Healthy-donor T-cells electroporated with in-vitro transcribed TCRα and β chains. (A) Flow cytometry plots of A*01:01/ILDTAGKEEY (SEQ ID NO: 1)-tetramer stained cells. Negative control - cells electroporated without mRNA ('Electroporated Nothing', EN) (B-C) Electroporated T-cells co-incubated overnight with peptide pulsed IHW01161 cells. (B) IFNγ ELISA; 10 μM peptide; one-way ANOVA, with Tukey's correction for multiple-comparisons; Error bars represent SEM of biological triplicates. (C) 4-1BB peptide-titration assay; 2-way ANOVA, with Sidak's correction for multiple-comparisons.

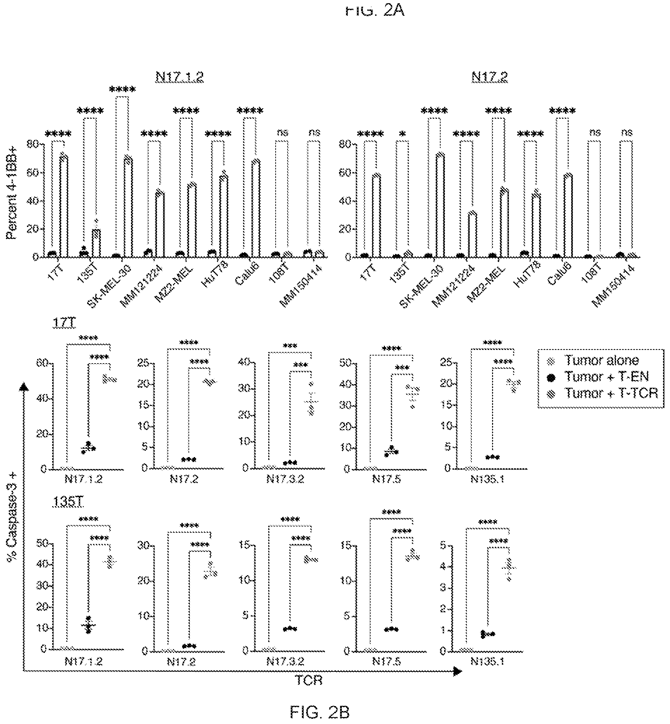

FIGs. 2A-B: Neoantigen-specific TCRs convey reactivity and cytotoxic capacity towards tumor cells which endogenously express the neoantigen

Healthy-donor (D3) T-cells electroporated with in-vitro transcribed TCR α and β chains and co-incubated with tumor-derived cell-lines. T-cell negative control: donor cells electroporated without mRNA ('Electroporated Nothing', EN). (A) percent T-cells expressing activation marker 4-1BB after overnight 1:1 co-incubation; 108T - A*01:01+/NRAS wild-type; MM150414 - A*01:01-/NRAS.Q61K; 2-way ANOVA with Sidak's correction for multiple-comparisons. (B) Cleaved caspase-3 killing assay: percent tumor cells expressing cleaved caspase-3 after 3-hour co-incubation at 3:1 effector to target ratio; one-way ANOVAs with Tukey's correction for multiple-comparisons. Error bars represent SEM of biological triplicates throughout.

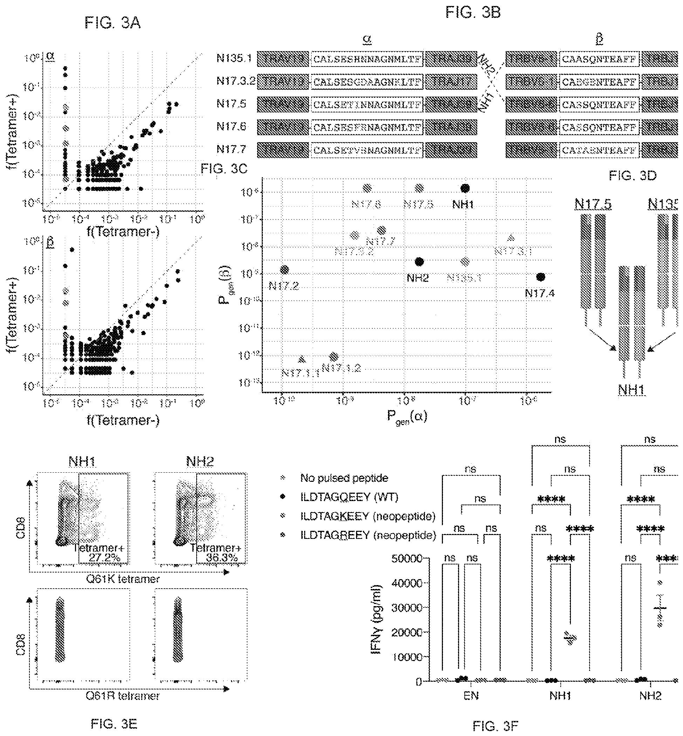

FIGs. 3A-F: Inspection of the 17TIL repertoire reveals a cluster of four TCRs which are similar to N135.1: N17.3.2, N17.5, N17.6 and N17.7. The top frequent TCRs of this list, N17.3.2 and N17.5, were validated as potent and neoantigen-specific. (A) Both α and β chains for the similarity cluster are enriched in the tetramer+ subpopulation. α/β pairings were confirmed by single-cell TCR-sequencing. Representative data from bulk TCR-sequencing replicate #1. (B) Sequence comparison of the TCRs' variable regions. Note Levenshtein distance of up to four amino-acids and V/J genes' similarity. (C) TCRs are plotted according to the probability of generation of their α (X axis) and β chains (Y axis). The N135.1 similarity cluster is encircled. NH1 (NRAS hybrid TCR #1) combines the most probable α/β within the similarity cluster; it is generated through chain swapping between N135.1 and N17.5. NH2 is NH1's counterpart, mixing NA17.5 with NB135.1. (D) Illustration of the chain swapping generating NH1 - combining NA135.1 with NB17.5. (E-F) Donor (D3) T-cells were electroporated with in-vitro transcribed TCR α and β chains, to express NH1 and NH2. T-cell negative control: donor cells electroporated without mRNA ('Electroporated Nothing', EN). (E) Flow cytometry analysis of CD8 and tetramer stained cells. (F) IFNg ELISA after overnight 1:1 co-incubation with IHW01161 presenting cells, pulsed with the wild-type or mutant peptides. IHW01161 without pulsed peptide (DMSO only) serve as negative-control. Error bars represent SEM of biological triplicates; 2-way ANOVA with Tukey's correction for multiple-comparisons.

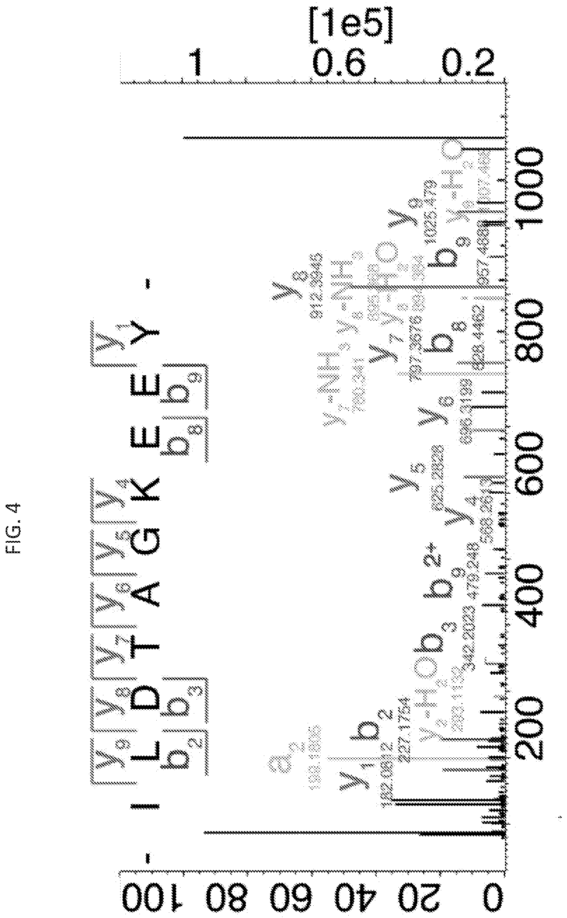

FIG. 4: Tandem mass spectra of ILDTAGKEEY (SEQ ID NO: 1) as it was identified in HLA-peptidomics of 17T. Method - FTMS; HCD, Score - 91.55, m/z - 569.79.

## DESCRIPTION OF SPECIFIC EMBODIMENTS OF THE INVENTION

The present invention is defined in the claims.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Immunotherapeutics have curative potential in metastatic cancer, as demonstrated specifically in melanoma. The anti-tumor effect is oftentimes mediated through T-cell recognition of neo-antigens; i.e., HLA-presented mutation-bearing peptides. With few exceptions, identified neo-antigens from responders stem from private mutations, and thus cannot be generalized beyond the individual patient. By definition, "recurrent neo-antigens" are such that are shared among groups of patients. Naturally, these are antigens that derive from common driver mutations and present on common HLA alleles. Moreover, due to the clonality of driver mutations, they are expected to present uniformly within tumors and across metastases. Recurrent neo-antigens should therefore have great clinical value, as they may serve for the development of effective, tumor-specific, "off-the-shelf" therapies.

Previously, the present inventors uncovered a new approach for the identification of recurrent neoantigens which combined a bioinformatic analysis which considers both the presence of recurrent mutations and the patient HLA allotype together with binding predictions (WO2020/234875). Using this approach they predicted that HLA-A*01:01/NRAS.Q61K hotspot neo-antigen is a robust immunogenic target that is relevant for thousands of patients.

The present inventors have now found a way to streamline this approach whereby an *in vitro* system is used to further reduce the number of potential candidate neoantigens prior to *in vivo* corroboration. This step is carried out following the bioinformatics analysis and binding prediction analysis described above and allows for a quicker and more efficient identification process. Since the *in-vivo* step can utilize expensive reagents (e.g. isotope labeled candidates which are spiked into the tumor sample for mass spectroscopy analysis), narrowing the list of potential candidates prior to the corroboration step has a great impact on the efficiency of the process.

Disclosed is a method of selecting a recurrent HLA-presented neoantigen which can be targeted in a cancer-immunotherapy treatment, the method comprising:

(a) analyzing the frequency of occurrence of a cancer-associated mutated protein in the context of an individual HLA allele in tumor cells of a plurality of cancer patients,; and

(b) determining the binding affinity of peptides of 8-14 amino acids in length derived from the cancer-associated mutated protein to the individual HLA allele, wherein the peptides comprise a mutation compared to the wild-type protein,

wherein a candidate peptide which binds with an affinity above a first predetermined level to an HLA allele having a frequency of occurrence above a second predetermined level, is selected as a candidate HLA-presented neoantigen that can be targeted in a cancer-immunotherapy treatment;

(c) corroborating that the candidate peptide is presented by the HLA allele in an *in vitro* expression system in antigen presenting cells; and

(d) confirming that the corroborated peptide is presented by the HLA allele in tumor cells of at least one cancer patient.

As used herein the term "neoantigen" is an epitope that has at least one alteration that makes it distinct from the corresponding wild-type, parental antigen, e.g., via mutation in a tumor cell or post-translational modification specific to a tumor cell. A neoantigen can include a polypeptide sequence or a nucleotide sequence. A mutation can include a frameshift or nonframeshift indel, missense or nonsense substitution, splice site alteration, genomic rearrangement or gene fusion, or any genomic or expression alteration giving rise to a neoORF. A mutation can also include a splice variant. Post-translational modifications specific to a tumor cell can include aberrant phosphorylation. Post-translational modifications specific to a tumor cell can also include a proteasome-generated spliced antigen.

In one embodiment, the neoantigen is a short peptide that is bound to a class I or II MHC receptor thus forming a ternary complex that can be recognized by a T-cell bearing a matching T-cell receptor binding to the MHC/peptide complex with appropriate affinity. Peptides binding to MHC class I molecules are typically about 8-14 amino acids in length. T-cell epitopes that bind to MHC class II molecules are typically about 12-30 amino acids in length. In the case of peptides that bind to MHC class II molecules, the same peptide and corresponding T cell epitope may share a common core segment, but differ in the overall length due to flanking sequences of differing lengths upstream of the amino-terminus of the core sequence and downstream of its carboxy terminus, respectively. A T-cell epitope may be classified as an antigen if it elicits an immune response.

Proteins from which the neoantigens are derived comprise cancer-associated modifications. Exemplary modifications include, but are not limited to cancer associated mutations and cancer-associated phosphorylation patterns.

The term "mutation" refers to a change of or difference in the nucleic acid sequence (nucleotide substitution, addition or deletion) compared to a reference. A "somatic mutation" can occur in any of the cells of the body except the germ cells (sperm and egg) and therefore are not passed on to children. These alterations can (but do not always) cause cancer or other diseases. Preferably a mutation is a non-synonymous mutation. The term "non-synonymous mutation" refers to a mutation, preferably a nucleotide substitution, which does result in an amino acid change such as an amino acid substitution in the translation product.

According to the invention, the term "mutation" includes point mutations, Indels, fusions, chromothripsis and RNA edits.

According to a specific embodiment, the mutation is a point mutation - i.e. a single amino acid substitution.

According to the invention, the term "Indel" describes a special mutation class, defined as a mutation resulting in a colocalized insertion and deletion and a net gain or loss in nucleotides. In coding regions of the genome, unless the length of an indel is a multiple of 3, they produce a frameshift mutation. Indels can be contrasted with a point mutation; where an Indel inserts and deletes nucleotides from a sequence, a point mutation is a form of substitution that replaces one of the nucleotides. In one embodiment, the indel is a frameshift deletion mutation. In another embodiment, the indel is a frameshift insertion mutation.

Fusions can generate hybrid genes formed from two previously separate genes. It can occur as the result of a translocation, interstitial deletion, or chromosomal inversion. Often, fusion genes are oncogenes. Oncogenic fusion genes may lead to a gene product with a new or different function from the two fusion partners. Alternatively, a proto-oncogene is fused to a strong promoter, and thereby the oncogenic function is set to function by an upregulation caused by the strong promoter of the upstream fusion partner. Oncogenic fusion transcripts may also be caused by trans-splicing or read-through events.

According to the invention, the term "chromothripsis" refers to a genetic phenomenon by which specific regions of the genome are shattered and then stitched together via a single devastating event.

According to the invention, the term "RNA edit" or "RNA editing" refers to molecular processes in which the information content in an RNA molecule is altered through a chemical change in the base makeup. RNA editing includes nucleoside modifications such as cytidine (C) to uridine (U) and adenosine (A) to inosine (I) deaminations, as well as non-templated nucleotide additions and insertions. RNA editing in mRNAs effectively alters the amino acid sequence of the encoded protein so that it differs from that predicted by the genomic DNA sequence.

Preferably, the mutations are non-synonymous mutations, preferably non-synonymous mutations of proteins expressed in a tumor or cancer cell.

In a particular embodiment, the protein which expresses a cancer-related modification pattern is expressed in melanoma cells, lung cancer cells, renal cancer cells or Head and neck squamous carcinoma cells.

Preferably, the protein which expresses a cancer-related modification pattern is expressed in melanoma cells.

Preferably, the protein which expresses a cancer-related modification pattern is a human protein.

Examples of proteins which may express cancer related modification patterns include those that are members of the RAS family - e.g. Neuroblastoma RAS Viral (V-Ras) Oncogene Homolog (NRAS; UniProtKB - P01111), Kirsten rat sarcoma viral oncogene homolog (KRAS; UniProtKB - P01116) and Harvey Rat Sarcoma Viral Oncogene Homolog (HRAS, UniProtKB - P01112).

Specific contemplated NRAS variants include Q61K, Q61R, Q61L and Q61H.

Another example of a cancer associated mutated protein is a RAF kinase - e.g. B-RAF UniProtKB - P15056.

Specific B-RAF variants include V600E, V600M, G466E, H725Y, K601E and V600G.

Other examples include, but are not limited to kallikrein 4, papillomavirus binding factor (PBF), preferentially expressed antigen of melanoma (PRAME), Wilms' tumor-1 (WT1), Hydroxysteroid Dehydrogenase Like 1 (HSDL1), mesothelin, cancer testis antigen (NY-ESO-1), carcinoembryonic antigen (CEA), p53, human epidermal growth factor receptor 2/neuro receptor tyrosine kinase (Her2/Neu), carcinoma-associated epithelial cell adhesion molecule EpCAM), ovarian and uterine carcinoma antigen (CA125), folate receptor a, sperm protein 17, tumor-associated differentially expressed gene-12 (TADG-12), mucin-16 (MUC-16), L1 cell adhesion molecule (L1CAM), mannan-MUC-1, Human endogenous retrovirus K (HERV-K-MEL), Kitakyushu lung cancer antigen-1 (KK-LC-1), human cancer/testis antigen (KM-HN-1), cancer testis antigen (LAGE-1), melanoma antigen-A1 (MAGE-A1), Sperm surface zona pellucida binding protein (Sp17), Synovial Sarcoma, X Breakpoint 4 (SSX-4), Transient axonal glycoprotein-1 (TAG-1), Transient axonal glycoprotein-2 (TAG-2), Enabled Homolog (ENAH), mammoglobin-A, NY-BR-1, Breast Cancer Antigen, (BAGE-1), B melanoma antigen, melanoma antigen-A1 (MAGE-A1), melanoma antigen-A2 (MAGE-A2), mucin k, synovial sarcoma, X breakpoint 2 (SSX-2), Taxol-resistance-associated gene-3 (TRAG-3), Avian Myelocytomatosis Viral Oncogene (c-myc), cyclin B1, mucin 1 (MUC1), p62, survivin, lymphocyte common antigen (CD45), Dickkopf WNT Signaling Pathway Inhibitor 1 (DKK1), telomerase, Kirsten rat sarcoma viral oncogene homolog (K-ras), G250, intestinal carboxyl esterase, alpha-fetoprotein, Macrophage Colony-Stimulating Factor (M-CSF), Prostate-specific membrane antigen (PSMA), caspase 5 (CASP-5), Cytochrome C Oxidase Assembly Factor 1 Homolog (COA-1), O-linked (3-N-acetylglucosamine transferase (OGT), Osteosarcoma Amplified 9, Endoplasmic Reticulum Lectin (OS-9), Transforming Growth Factor Beta Receptor 2 (TGF-betaRII), murine leukemia glycoprotein 70 (gp70), Calcitonin Related Polypeptide Alpha (CALCA), Programmed cell death 1 ligand 1 (CD274), Mouse Double Minute 2Homolog (mdm-2), alpha-actinin-4, elongation factor 2, Malic Enzyme 1 (ME1), Nuclear Transcription Factor Y Subunit C (NFYC), G Antigen 1,3 (GAGE-1,3), melanoma antigen-A6 (MAGE-A6), cancer testis antigen XAGE-1b, six transmembrane epithelial antigen of the prostate 1 (STEAP1), PAP, prostate specific antigen (PSA), Fibroblast Growth Factor 5 (FGF5), heat shock protein hsp70-2, melanoma antigen-A9 (MAGE-A9), Arg-specific ADP-ribosyltransferase family C (ARTC1), B-Raf Proto-Oncogene (B-RAF), Serine/Threonine Kinase, beta-catenin, Cell Division Cycle 27 homolog (Cdc27), cyclin dependent kinase 4 (CDK4), cyclin dependent kinase 12 (CDK12), Cyclin Dependent Kinase Inhibitor 2A (CDKN2A), Casein Kinase 1 Alpha 1 (CSNK1A1), Fibronectin 1 (FN1), Growth Arrest Specific 7 (GAS7), Glycoprotein nonmetastatic melanoma protein B (GPNMB), HAUS Augmin Like Complex Subunit 3 (HAUS3), LDLR-fucosyltransferase, Melanoma Antigen Recognized By T-Cells 2 (MART2), myostatin (MSTN), Melanoma Associated Antigen (Mutated) 1 (MUM-1-2-3), Poly(A) polymerase gamma (neo-PAP), myosin class I, Protein phosphatase 1 regulatory subunit 3B (PPP1R3B), Peroxiredoxin-5 (PRDXS), Receptor-type tyrosine-protein phosphatase kappa (PTPRK), Transforming protein N-Ras (N-ras), retinoblastoma-associated factor 600 (RBAF600), sirtuin-2 (SIRT2), SNRPD1, triosephosphate isomerase, Ocular Albinism Type 1 Protein (OA1), member RAS oncogene family (RAB38), Tyrosinase related protein 1-2 (TRP-1-2), Melanoma Antigen Gp75 (gp75), tyrosinase, Melan-A (MART-1), Glycoprotein 100 melanoma antigen (gp100), N-acetylglucosaminyltransferase V gene (GnTVf), Lymphocyte Antigen 6 Complex Locus K (LY6K), melanoma antigen-A10 (MAGE-A10), melanoma antigen-A12 (MAGE-A12), melanoma antigen-C2 (MAGE-C2), melanoma antigen NA88-A, Taxol-resistant-associated protein 3 (TRAG-3), BDZ binding kinase (pbk), caspase 8 (CASP-8), sarcoma antigen 1 (SAGE), Breakpoint Cluster Region-Abelson oncogene (BCR-ABL), fusion protein in leukemia, dek-can, Elongation Factor Tu GTP Binding Domain Containing 2 (EFTUD2), ETS Variant gene 6/acute myeloid leukemia fusion protein (ETV6-AML1), FMS-like tyrosine kinase-3 internal tandem duplications (FLT3-ITD), cyclin-A1, Fibronectin Type III Domain Containing 3B (FDNC3B,) promyelocytic leukemialretinoic acid receptor alpha fusion protein (pml-RARalpha), melanoma antigen-C1 (MAGE-C1), membrane protein alternative spliced isoform (D393-CD20), melanoma antigen-A4 (MAGE-A4), or melanoma antigen-A3 (MAGE-A3).

Additional examples of proteins that may express cancer related modification patterns are known in the art and are described, for example, in Reuschenbach et al., Cancer Immunol. Immunother. 58:1535-1544 (2009); Parmiani et al., J. Nat. Cancer Inst. 94:805-818 (2002); Zarour et al., Cancer Medicine. (2003); Bright et al., Hum. Vaccin. Immunother. 10:3297-3305 (2014); Wurz et al., Ther. Adv. Med. Oncol. 8:4-31 (2016); Criscitiello, Breast Care 7:262-266 (2012); Chester et al., J. Immunother. Cancer 3:7 (2015); Li et al., Mol. Med. Report 1:589-594 (2008); Liu et al., J. Hematol. Oncol. 3:7 (2010); Bertino et al., Biomed. Res. Int. 731469 (2015); and Suri et al., World J. Gastrointest. Oncol. 7:492-502 (2015).

In one embodiment, the mutations are cancer specific somatic mutations.

Methods for detecting sequence alteration are well known in the art and include, but not limited to, DNA sequencing, electrophoresis, an enzyme-based mismatch detection assay and a hybridization assay such as PCR, RT-PCR, RNase protection, in-situ hybridization, primer extension, Southern blot, Northern Blot and dot blot analysis.

Sequence alterations in a specific gene can also be determined at the protein level using e.g. chromatography, electrophoretic methods, immunodetection assays such as ELISA and Western blot analysis and immunohistochemistry.

In one embodiment, the step of identifying cancer specific somatic mutations or identifying sequence differences involves using next generation sequencing (NGS).

In one embodiment, the step of identifying cancer specific somatic mutations or identifying sequence differences comprises sequencing genomic DNA and/or RNA of the tumor specimen.

To reveal cancer specific somatic mutations or sequence differences the sequence information obtained from the tumor specimen is preferably compared with a reference such as sequence information obtained from sequencing nucleic acid such as DNA or RNA of normal non-cancerous cells such as germline cells which may either be obtained from the patient or a different individual. In one embodiment, normal genomic germline DNA is obtained from peripheral blood mononuclear cells (PBMCs).

The term "genome" relates to the total amount of genetic information in the chromosomes of an organism or a cell.

The term "exome" refers to part of the genome of an organism formed by exons, which are coding portions of expressed genes. The exome provides the genetic blueprint used in the synthesis of proteins and other functional gene products. It is the most functionally relevant part of the genome and, therefore, it is most likely to contribute to the phenotype of an organism. The exome of the human genome is estimated to comprise 1.5 % of the total genome (Ng, P C et al., PLoS Gen., 4(8): 1-15, 2008).

The term "transcriptome" relates to the set of all RNA molecules, including mRNA, rRNA, tRNA, and other non-coding RNA produced in one cell or a population of cells. In context of the present invention the transcriptome means the set of all RNA molecules produced in one cell, a population of cells, preferably a population of cancer cells, or all cells of a given individual at a certain time point.

According to the invention, a "reference" may be used to correlate and compare the results obtained in the methods of the invention from a tumor specimen. Typically the "reference" may be obtained on the basis of one or more normal specimens, in particular specimens which are not affected by a cancer disease, either obtained from a patient or one or more different individuals, preferably healthy individuals, in particular individuals of the same species. A "reference" can be determined empirically by testing a sufficiently large number of normal specimens.

Any suitable sequencing method can be used according to the invention for determining mutations, Next Generation Sequencing (NGS) technologies being preferred. Third Generation Sequencing methods might substitute for the NGS technology in the future to speed up the sequencing step of the method. For clarification purposes: the terms "Next Generation Sequencing" or "NGS" in the context of the present invention mean all novel high throughput sequencing technologies which, in contrast to the "conventional" sequencing methodology known as Sanger chemistry, read nucleic acid templates randomly in parallel along the entire genome by breaking the entire genome into small pieces.

Methods for identifying disease-specific phosphorylation patterns are known in the art and include for example stable isotope labeling with amino acids in cell culture (SILAC), RRPA, and phospho-specific Western blots.

In a preferred embodiment, the HLA allele is a class I HLA allele. In particular embodiments, the class I HLA allele is an HLA-A allele or an HLA-B allele. In a preferred embodiment, the HLA allele is a class II HLA allele. Sequences of class I and class II HLA alleles can be found in the IPD-EVIGT/HLA Database. Exemplary HLA alleles include but are not limited to A*01:01, A*02:01, A*02:03, A*02:04, A*02:07, A*03:01, A*24:02, A*29:02, A*31:01, A*68:02, B*35:01, B*44:02, B*44:03, B*51 :01, B*54:01 or B57:01 In particular embodiments, the HLA allele is HLA-A*01:01.

Subject specific HLA alleles or HLA genotype of a subject may be determined by any method known in the art. In a particular embodiment, HLA genotypes are determined by any method described in International Patent Application number PCT/US2014/068746, published June 11, 2015 as WO2015085147. Briefly, the methods include determining polymorphic gene types that may comprise generating an alignment of reads extracted from a sequencing data set to a gene reference set comprising allele variants of the polymorphic gene, determining a first posterior probability or a posterior probability derived score for each allele variant in the alignment, identifying the allele variant with a maximum first posterior probability or posterior probability derived score as a first allele variant, identifying one or more overlapping reads that aligned with the first allele variant and one or more other allele variants, determining a second posterior probability or posterior probability derived score for the one or more other allele variants using a weighting factor, identifying a second allele variant by selecting the allele variant with a maximum second posterior probability or posterior probability derived score, the first and second allele variant defining the gene type for the polymorphic gene, and providing an output of the first and second allele variant.

Preferably a cancer-associated mutated protein in the context of an individual HLA allele is selected which has a high frequency in a predetermined number of cancer patients (e.g. at least greater than 200, 300, 400, 500, 600, 700, 800, 900, 1000, 2000 or more).

The cancer patient group may be suffering from the same cancer type - melanoma or may be part of a pan-cancer group suffering from a number of different cancer types.

The cancer patient group may include melanoma patients, thyroid cancer patients, pheochromocytoma patients, seminoma patients, stomach adenocarcinoma patients, cholangiocarcinoma patients, pancreatic adenocarcinoma patients, colorectal adenocarcinoma, leukemia patients, bladder urothelial carcinoma patients, endometrial carcinoma patients, thymic epithelial tumor patients, non-small cell lung cancer patients, sarcoma patients, ovarian cancer patients and prostate cancer patients, or any combination of the above described cancer patients.

In one embodiment, the cancer patient group includes only melanoma cancer patients.

In another embodiment, the cancer patient group comprises those that have shown to be resistant to a particular therapy.

It will be appreciated that the HLA status may have a high frequency in the group and/or there is a high frequency of the presence of the particular mutation in that group. Preferably, the HLA status frequency is high (e.g. over 0.5 %, 1 %, 2 %, 3 %, 4 %, 5 %, 6 %, 7 %, 8 %, 9 %, 10 %) and the frequency of the particular mutation in that group is also high (e.g. over 0.5 %, 1 %, 2 %, 3 %, 4 %, 5 %, 6 %, 7 %, 8 %, 9 %, 10 %).

In a particular embodiment, the proportion of tumor cells harboring the cancer-associated mutated protein (i.e. clonality) is also taken into account during the initial screening stage. Thus, a candidate peptide which binds with an affinity above a first predetermined level to an HLA allele having a frequency of occurrence above a second predetermined level, and which is harbored in a proportion of tumor cells above a third predetermined level is selected as a candidate HLA-presented neoantigen that can be targeted in a cancer-immunotherapy treatment.

In one embodiment, the measure of clonality is the variant allele frequency (VAF).

In one embodiment, the variant allele frequency is the sum of detected sequences, in particular reads, covering the mutation site and carrying the mutation divided by the sum of all detected sequences, in particular reads, covering the mutation site. In one embodiment, the variant allele frequency is the sum of mutated nucleotides at the mutation site divided by the sum of all nucleotides determined at the mutation site. In one embodiment, for ascertaining a score for ascertaining the proportion of tumor cells harboring the cancer-associated mutated protein, a score for the level of expression of the protein to which the modification is associated is multiplied with a score for the frequency of the modified protein among the protein to which the modification is associated.

In general, the value of a variant allele frequency can be from 0 to 1, where a variant allele frequency of 0 indicates no sequence reads that possess the alternate allele at the position and where a variant allele frequency of 1 indicates that all sequence reads possess the alternate allele at the position. In other embodiments, other ranges and/or values of variant allele frequencies can be used.

Thus, for example, in one embodiment, when the mean VAF is between 0.1-0.55 in the tumor sample, the mutation of the protein is considered. In another embodiment, when the media VAF is between 0.1-0.55 in the tumor sample, the mutation of the protein is considered.

Once the HLA allele and the particular mutated protein have been selected, the binding affinity of peptides (which comprise the selected mutation) of 8-14 amino acids in length derived from the selected cancer-associated mutated protein to the selected HLA allele are analyzed.

Methods of analyzing binding affinity of peptides to HLA alleles are known in the art.

In one embodiment, the binding affinity can be predicted using a prediction algorithm for HLA binding. Such prediction algorithms include but are not limited to NetMHC, NetMHC **II,** NetMHCpan, IEDB Analysis Resource (URL immuneepitope(dot)org), RankPep, PREDEP, SVMHC, Epipredict, HLABinding, and others (see e.g., J Immunol Methods 2011; 374:1-4).

Using such predictions, a list of candidate neoantigens can be generated that bind with an affinity above a predetermined amount to the HLA. According to a particular embodiment, only candidate peptides that bind with a %Rank ≤0.5 (default parameters of NetMHCpan) are selected, or a corresponding level using a different prediction algorithm. According to another embodiment, candidate peptides are selected whose binding can be characterized as %Rank ≤2 (default parameters of NetMHCpan), or a corresponding level using a different prediction algorithm.

It will be appreciated that the binding affinity may be lower than the above mentioned levels if the HLA allele frequency and/or the frequency of the mutation is high.

Thus, it will be understood that it is the combination of the three parameters - frequency of HLA allele, frequency of occurrence of the mutation and the binding affinity which together dictate the selection of candidate neoantigens, and is not based on only a single parameter. Thus, the predetermined amount for any one of the parameters is not a fixed amount but rather is fluid and can be changed according to the levels of the other two parameters.

Upon determination of candidate neoantigens using the above described steps, the candidate neoantigens are tested in an in-vitro system in order to further narrow down the list of potential candidates.

The in-vitro system typically comprises antigen presenting cells, which are genetically modified to express the candidate neoantigen and its cognate HLA.

Antigen presenting cells (APC) are cells which present peptide fragments of protein antigens in association with HLA (MHC) molecules on their cell surface.

Examples of APCs include, but are not limited to dendritic cells, macrophages, Langerhans cells and B cells.

According to a particular embodiment, the APCs are dendritic cells or B cells. Most preferable are B cells.

In one embodiment, the APCs are immortalized - i.e. a transformed cell line, such as Epstein Barr Virus (EBV)-transformed B cells.

In one embodiment, the APCs are genetically modified to express the particular HLA allele which has been predicted to bind the candidate neoantigen.

B cells that are HLA deficient (e.g. B721.221) can be used so that the system is "clean" from non-relevant HLAs.

An exemplary method for deleting/inactivating endogenous class I or class II genes in antigen presenting cells which express non-relevant HLA alleles is CRISPR-Cas9 mediated genome editing.

Typically the candidate peptide is expressed in the cell using a 25-27 mer minigene system and/or a 45-47 mer minigene system.

In this way, extended peptides of various lengths surrounding the identified mutation are expressed in the cell. Upon expression, the endogenous proteasomal system of the cell digests the extended peptide into shorter lengthed peptides, such that the neoantigen is presented on the surface of the cell in the context of the HLA.

The minigene comprises a nucleic acid molecule encoding the extended neoantigen. In any of the aforementioned embodiments, the expression construct may comprise tandem minigenes, wherein each minigene comprises a nucleic acid molecule encoding a neoantigen. For example, the tandem minigenes may comprise nucleic acid molecules encoding from two to about 20 neoantigens, or from two to about 10 neoantigens, or from two to about five neoantigens.

Using an in vitro system ensures that the neoantigen is expressed in much higher levels compared to endogenous amounts and it is therefore easier to identify the particular peptide that is presented by the HLA using mass spectrometry and other comparable techniques, including but not limited to thin layer chromatography, electrophoresis, in particular capillary electrophoresis, solid phase extraction (CSPE), reverse-phase high performance liquid chromatography, amino-acid analysis after acid hydrolysis and by fast atom bombardment (FAB) mass spectrometric analysis, as well as MALDI and ESI-Q-TOF mass spectrometric analysis.

In a particular embodiment, the analysis may be determined using liquid chromatography and tandem mass spectrometry (LC-MS/MS) and/or HPLC - see for example Kalaora et al., Oncotarget. 2016 Feb 2; 7(5): 5110-5117, the contents of which being incorporated herein by reference.

Upon confirmation that the candidate neoantigen is presented by the cognate HLA, the candidate peptide and HLA pair is corroborated using tumor cells (e.g. fresh tumor cells, PDX or tumor cell lines). According to a particular embodiment, the candidate peptide and the HLA pair is corroborated in an in-vivo system using tumor cells derived from a cancer patient known to have the relevant HLA status.

Presentation of the neoantigen by the specified HLA is corroborated in the tumor cells using methods known in the art. For example, peptides can be acid-eluted from tumor cells or from HLA molecules that are immunoprecipitated from tumor, and then identified using mass spectrometry. During the identification process, a spiked-in heavy peptide (the candidate neoantigen) may be introduced into the system serving as a control. This also serves to allow for quantification of the neoantigen in the tumor sample.

The reactivity of the selected neoantigens can then be assessed as further described herein below.

In one embodiment, the candidate peptides are loaded onto the APCs under conditions that allow them to be presented on the surface of the APCs.

To be presented on the surface of the APCs, they have to cross the APC cell membrane and loaded onto newly synthesized HLA class I or II receptors. Formed HLA-peptide complexes are translocated onto the cell membrane, where they are readily available for T-cell recognition.

In one embodiment, the peptides are incubated with the APCs in a medium which maintains the APCs in a viable state (e.g. RPMI) for an amount of time between 12-48 hours, 12-24 hours, 6-48 hours or 8-48 hours. The concentration of the peptide is preferably between 10-50 $\mu$M and more preferably between 10-30 $\mu$M during the loading stage.

Next, activation of CD4+ or CD8+ T cells may be determined. Methods for detecting specific T cell activation include detecting the proliferation of T cells, the production of cytokines (e.g., lymphokines, interferon gamma, TNF alpha), or the generation of cytolytic activity. For CD4+ T cells, a preferred method for detecting specific T cell activation is the detection of the proliferation of T cells. For CD8+ T cells, a preferred method for detecting specific T cell activation is the detection of the generation of cytolytic activity.

According to a particular embodiment, in order to determine the reactivity of the peptides, an ELISPOT assay may be carried out, where the CD8+ CTL response, which can be assessed by measuring IFN-gamma production by antigen-specific effector cells, is quantitated by measuring the number of Spot Forming Units (SFU) under a stereomicroscope (Rininsland et al., (2000) J Immunol Methods: 240(1-2):143-155.). In this assay, antigen-presenting cells (APC) are immobilized on the plastic surface of a micro titer well, and effector T cells are added at various effector:target ratios. Antigen presenting cells are preferably B cells or dendritic cells. The binding of APC's by antigen-specific effector cells triggers the production of cytokines including IFN-gamma by the effector cells (Murali-Krishna et al., (1998) Adv Exp Med Biol.: 452:123-142). In one embodiment subject specific T cells are used in the ELISPOT assay. The amount of soluble IFNy secreted from the TILs may also be measured by ELISA assay (e.g. Biolegend).

Another method for determining the reactivity of the peptides is by direct determination of cell lysis as measured by the classical assay for CTL activity namely the chromium release assay (Walker et al., (1987) Nature: 328:345-348;

Scheibenbogen et al., (2000) J Immunol Methods: 244(1-2):81-89.). Effector Cytotoxic T Lymphocytes (CTL) bind targets bearing antigenic peptide on Class I MHC and signal the targets to undergo apoptosis. If the targets are labeled with $^{51}$Chromium before the CTL are added, the amount of $^{51}$Cr released into the supernatant is proportional to the number of targets killed. Antigen-specific lysis is calculated by comparing lysis of target cells expressing disease or control antigens in the presence or absence of patient effector cells, and is usually expressed as the %-specific lysis. Percent specific cytotoxicity is calculated by (specific release-spontaneous release)/(maximum release-spontaneous release) and may be 20%-85% for a positive assay. Percent specific cytotoxicity is usually determined at several ratios of effector (CTL) to target cells (E:T). Additionally, the standard lytic assay is qualitative and must rely on a limiting dilution analysis (LDA) for quantitative results, and the LDA frequently underestimates the true level of CTL response. Although CTL can each kill many targets in vivo, in vitro this assay requires numbers of CTL equal to or greater than the number of targets for detectable killing. In one embodiment CTL responses are measured by the chromium release assay, monitoring the ability of T cells (Effector cells) to lyse radiolabelled HLA matched "target cells" that express the appropriate antigen-MHC complex.

As mentioned herein above, the present inventors previously predicted that HLA-A*01:01/NRAS.Q61K hotspot neo-antigen is a robust immunogenic target that is relevant for thousands of patients yearly - WO2020/234875. The present inventors have now identified a T cell receptor which is highly reactive to this HLA-presented neoantigen.

Specifically, this TCR was shown to recognize both the ILDTAGKEEY (SEQ ID NO: 1) and the ILDTAGREEY (SEQ ID NO: 34) neopeptide at concentrations as low as 1nM and to induce 4-1BB upregulation upon co-incubation with relevant cancer cell-lines (e.g. melanoma cell lines and lung adenocarcinoma cell lines).

Thus, according to a first aspect of the present invention, there is provided a population of T cells for use in treating cancer, wherein an alpha chain of a TCR of at least 10 % of the T cells of the population has a CDR3 amino acid sequence as set forth in SEQ ID NO: 6, and a beta chain of the CDR has a CDR3 amino acid sequence as set forth in SEQ ID NO: 7.

Examples of cancers include but are not limited to melanoma, colon cancer, breast cancer, thyroid cancer, stomach cancer, colorectal cancer, leukemia cancer, bladder cancer, lung cancer, ovarian cancer, breast cancer and prostate cancer.

In one embodiment, the cancer is a RAS-associated cancer, such as Oncogene Homolog (NRAS; UniProtKB - P01111) associated cancer - e.g. Q61K, Q61R NRAS.

In one embodiment, the cancer is a metastasized cancer.

T cells used to treat the cancers described herein may be derived from subjects that are suffering from the cancer (adoptive cell therapy - ACT). In one embodiment, the T cells are derived from peripheral blood lymphocytes of the subject.

In one embodiment, the T cells are autologous T cells.

In another embodiment, the T cells are non-autologous T cells.

ACT refers to the transfer of cells, most commonly immune-derived cells, back into the same patient or into a new recipient host with the goal of transferring the immunologic functionality and characteristics into the new host. If possible, use of autologous cells helps the recipient by minimizing GVHD issues. The adoptive transfer of autologous tumor infiltrating lymphocytes (TIL) (Besser et al., (2010) Clin. Cancer Res 16 (9) 2646-55; Dudley et al., (2002) Science 298 (5594): 850-4; and Dudley et al., (2005) Journal of Clinical Oncology 23 (10): 2346-57) or genetically re-directed peripheral blood mononuclear cells (Johnson et al., (2009) Blood 114 (3): 535-46; and Morgan et al., (2006) Science 314(5796) 126-9) has been used to successfully treat patients with advanced solid tumors, including melanoma and colorectal carcinoma, as well as patients with CD19-expressing hematologic malignances (Kalos et al., (2011) Science Translational Medicine 3 (95): 95ra73). In one embodiment TCRs are selected for administering to a subject based on binding to neoantigens as identified herein. In one embodiment T cells are expanded using methods known in the art. Expanded T cells that express tumor specific TCRs may be administered back to a subject. In another embodiment PBMCs are transduced or transfected with polynucleotides for expression of TCRs and administered to a subject. T cells expressing TCRs specific to neoantigens are expanded and administered back to a subject.

The T cell populations expressing T cell receptors on the surface thereof bind to at least one of the peptide epitopes having the sequences as set forth in SEQ ID NOs: 1 and 34 and have antigenic specificity towards the corresponding peptides. In one embodiment, the T cells have antigenic specificity towards both the peptide epitopes as set forth in SEQ ID NOs: 1 and 34.

The phrase "antigenic specificity," as used herein, means that the TCR can specifically bind to and immunologically recognize mutated target, e.g., mutated NRAS or BRAF, with high avidity. For example, a TCR may be considered to have "antigenic specificity" for a mutated target if T cells expressing the TCR secrete at least about 200 pg/mL or more (e.g., 200 pg/mL or more, 300 pg/mL or more, 400 pg/mL or more, 500 pg/mL or more, 600 pg/mL or more, 700 pg/mL or more, 1000 pg/mL or more, 5,000 pg/mL or more, 7,000 pg/mL or more, 10,000 pg/mL or more, 20,000 pg/mL or more, or a range defined by any two of the foregoing values) of IFN-gamma upon co-culture with (a) antigen-negative HLA-A*01:01$^{+}$ target cells pulsed with a low concentration of mutated target peptide having a sequence as set forth in SEQ ID NO: 1 or 34 (e.g., about 0.05 ng/mL to about 5 ng/mL, 0.05 ng/mL, 0.1 ng/mL, 0.5 ng/mL, 1 ng/mL, 5 ng/mL, or a range defined by any two of the foregoing values or (b) antigen-negative HLA-A*01:01$^{+}$ target cells into which a nucleotide sequence encoding the mutated target peptide having a sequence as set forth in SEQ ID NO: 1 or 34 has been introduced such that the target cell expresses the mutated target. Cells expressing the inventive TCRs may also secrete IFN-gamma upon co-culture with antigen-negative HLA-A*01:01⁺ target cells pulsed with higher concentrations of mutated target peptide.

Alternatively or additionally, a TCR may be considered to have "antigenic specificity" for a mutated target if T cells expressing the TCR secrete at least twice as much IFN-gamma upon co-culture with (a) antigen-negative HLA-A*01:01⁺ target cells pulsed with a low concentration of mutated target peptide or (b) antigen-negative HLA-A*01:01⁺ target cells into which a nucleotide sequence encoding the mutated target has been introduced such that the target cell expresses the mutated target as compared to the amount of IFN-gamma expressed by a negative control. The negative control may be, for example, (i) T cells expressing the TCR, co-cultured with (a) antigen-negative HLA-A*01:01⁺ target cells pulsed with the same concentration of an irrelevant peptide (e.g., some other peptide with a different sequence from the mutated target peptide) or (b) antigen-negative HLA-A*01:01⁺ target cells into which a nucleotide sequence encoding an irrelevant peptide has been introduced such that the target cell expresses the irrelevant peptide, or (ii) non-transduced T cells (e.g., derived from PBMC, which do not express the TCR) co-cultured with (a) antigen-negative HLA-A*01:01⁺ target cells pulsed with the same concentration of mutated target peptide or (b) antigen-negative HLA-A*01:01⁺ target cells into which a nucleotide sequence encoding the mutated target has been introduced such that the target cell expresses the mutated target. IFN-gamma secretion may be measured by methods known in the art such as, for example, enzyme-linked immunosorbent assay (ELISA).

Alternatively or additionally, a TCR may be considered to have "antigenic specificity" for a mutated target if at least twice as many of the numbers of T cells expressing the TCR secrete IFN-gamma upon co-culture with (a) antigen-negative HLA-A*01:01⁺ target cells pulsed with a low concentration of mutated target peptide or (b) antigen-negative HLA-A*01:01⁺ target cells into which a nucleotide sequence encoding the mutated target has been introduced such that the target cell expresses the mutated target as compared to the numbers of negative control T cells that secrete IFN-gamma. The concentration of peptide and the negative control may be as described herein with respect to other aspects of the invention. The numbers of cells secreting IFN-gamma may be measured by methods known in the art such as, for example, ELISPOT.

Methods of engineering T cells to express recombinant T cell receptors for cancer treatment are disclosed in Ping et al Protein Cell. 2018 Mar; 9(3): 254-266.

The invention provides T cells expressing TCRs comprising two polypeptides (i.e., polypeptide chains), such as an alpha (alpha) chain of a TCR, a beta chain of a TCR, a gamma (gamma) chain of a TCR, a delta (delta) chain of a TCR, or a combination thereof. The polypeptides of the inventive TCR can comprise any amino acid sequence, provided that the TCR has antigenic specificity for the mutated target, e.g., mutated NRAS.

In an embodiment of the invention, the TCR comprises two polypeptide chains, each of which comprises a variable region comprising a complementarity determining region (CDR)1, a CDR2, and a CDR3 of a TCR.

As well as CDRs, the TCRs disclosed herein also comprise V regions and J regions. Particular combinations of V and J regions are presented in Table 1, herein below.

The sequence of a CDR3 region of an exemplary β chain of a T cell receptor which may be used according to this aspect of the present invention is set forth in SEQ ID NO: 7.

The sequence of a CDR3 region of an exemplary α chain of a T cell receptor which may be used according to this aspect of the present invention is set forth in SEQ ID NO: 6.

The present invention contemplates T cell populations, wherein at least 10 % , 20 %, 30 %, 40 %, 50 %, 60 %, 70 %, 80 %, 90 % of the T cells of the populations express the T cell receptor having a β chain CDR3 amino acid sequence as set forth in SEQ ID NO: 7 and an α chain CDR3 amino acid sequence as set forth in SEQ ID NO: 6.

In one embodiment at least 95 % of the T cells of the population express the T cell receptor having a β chain CDR3 amino acid sequence as set forth in SEQ ID NO: 7 and an α chain CDR3 amino acid sequence as set forth in SEQ ID NO: 6.

The present inventors further contemplate CAR-T cells - i.e. T cells expressing chimeric antibodies having a CDR3 amino acid sequence as set forth in SEQ ID NOs: 6 and 7.

It will be appreciated that the sequences of the CDR3 regions may comprise at least one or even two amino acid substitutions and retain binding activity.

In one embodiment, the amino acid substitution is a conservative substitution.

The term "conservative substitution" as used herein, refers to the replacement of an amino acid present in the native sequence in the peptide with a naturally or non-naturally occurring amino or a peptidomimetics having similar steric properties. Where the side-chain of the native amino acid to be replaced is either polar or hydrophobic, the conservative substitution should be with a naturally occurring amino acid, a non-naturally occurring amino acid or with a peptidomimetic moiety which is also polar or hydrophobic (in addition to having the same steric properties as the side-chain of the replaced amino acid).

As naturally occurring amino acids are typically grouped according to their properties, conservative substitutions by naturally occurring amino acids can be easily determined bearing in mind the fact that in accordance with the invention replacement of charged amino acids by sterically similar non-charged amino acids are considered as conservative substitutions.

For producing conservative substitutions by non-naturally occurring amino acids it is also possible to use amino acid analogs (synthetic amino acids) well known in the art. A peptidomimetic of the naturally occurring amino acid is well documented in the literature known to the skilled practitioner.

When affecting conservative substitutions the substituting amino acid should have the same or a similar functional group in the side chain as the original amino acid.

The phrase "non-conservative substitutions" as used herein refers to replacement of the amino acid as present in the parent sequence by another naturally or non-naturally occurring amino acid, having different electrochemical and/or steric properties. Thus, the side chain of the substituting amino acid can be significantly larger (or smaller) than the side chain of the native amino acid being substituted and/or can have functional groups with significantly different electronic properties than the amino acid being substituted. Examples of non-conservative substitutions of this type include the substitution of phenylalanine or cycohexylmethyl glycine for alanine, isoleucine for glycine, or -NH-CH[(-CH$_2$)$_{5-}$COOH]-CO- for aspartic acid. Those non-conservative substitutions which fall under the scope of the present invention are those which still constitute a peptide having anti-bacterial properties.

According to a particular embodiment, the TCR receptor comprises an alpha chain which comprises a CDR3 region as set forth in SEQ ID NO: 6 and a beta chain which comprises a CDR3 region as set forth in SEQ ID NO: 7.

Also contemplated are isolated antibodies and/or diabodies which comprise at least one of the CDR sequences specified herein.

The TCRs (and antibodies) of the invention of the invention can comprise synthetic amino acids in place of one or more naturally-occurring amino acids. Such synthetic amino acids are known in the art, and include, for example, aminocyclohexane carboxylic acid, norleucine, alpha-amino n-decanoic acid, homoserine, S-acetylaminomethyl-cysteine, trans-3- and trans-4-hydroxyproline, 4-aminophenylalanine, 4-nitrophenylalanine, 4-chlorophenylalanine, 4-carboxyphenylalanine, beta-phenylserine beta-hydroxyphenylalanine, phenylglycine, alpha-naphthylalanine, cyclohexylalanine, cyclohexylglycine, indoline-2-carboxylic acid, 1,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, alpha-aminocyclopentane carboxylic acid, alpha-aminocyclohexane carboxylic acid, alpha-aminocycloheptane carboxylic acid, .alpha.-(2-amino-2-norbornane)-carboxylic acid, alpha, gamma-diaminobutyric acid, alpha, beta-diaminopropionic acid, homophenylalanine, and alpha-tert-butylglycine.

The TCRs (and antibodies) of the invention (including functional variants thereof) can be glycosylated, amidated, carboxylated, phosphorylated, esterified, N-acylated, cyclized via, e.g., a disulfide bridge, or converted into an acid addition salt and/or optionally dimerized or polymerized, or conjugated.

The TCRs (and antibodies) of the invention can be obtained by methods known in the art such as, for example, de novo synthesis. Also, TCRs can be recombinantly produced using the nucleic acids described herein using standard recombinant methods. See, for instance, Green and Sambrook, Molecular Cloning: A Laboratory Manual, 4.sup.th ed., Cold Spring Harbor Press, Cold Spring Harbor, N.Y. (2012). Alternatively, the TCRs, polypeptides, and/or proteins described herein (including functional variants thereof) can be commercially synthesized by companies, such as Synpep (Dublin, Calif.), Peptide Technologies Corp. (Gaithersburg, Md.), and Multiple Peptide Systems (San Diego, Calif.). In this respect, the inventive TCRs, can be synthetic, recombinant, isolated, and/or purified. Included in the scope of the invention are conjugates, e.g., bioconjugates, comprising any of the inventive TCRs. Conjugates, as well as methods of synthesizing conjugates in general, are known in the art.

The populations of tumor-reactive T cells expressing subject-specific TCRs may be combined with a pharmaceutically acceptable carrier to obtain a pharmaceutical composition comprising a personalized cell population of tumor-reactive T cells. Preferably, the carrier is a pharmaceutically acceptable carrier. With respect to pharmaceutical compositions, the carrier can be any of those conventionally used for the administration of cells. Such pharmaceutically acceptable carriers are well-known to those skilled in the art and are readily available to the public. It is preferred that the pharmaceutically acceptable carrier be one which has no detrimental side effects or toxicity under the conditions of use. A suitable pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, Ill.), PLASMA-LYTE A (Baxter, Deerfield, Ill.), about 5% dextrose in water, or Ringer's lactate. In an embodiment, the pharmaceutically acceptable carrier is supplemented with human serum albumen.

The T cells can be administered by any suitable route as known in the art. Preferably, the T cells are administered as an intra-arterial or intravenous infusion, which preferably lasts approximately 30-60 min. Other examples of routes of administration include intraperitoneal, intrathecal and intralymphatic. T cells may also be administered by injection. T cells may be introduced at the site of the tumor.

The T cells described herein may be autologous to the subject or non-autologous.

For purposes of the invention, the dose, e.g., number of cells in the inventive cell population expressing subject specific TCRs, administered should be sufficient to effect, e.g., a therapeutic or prophylactic response, in the subject over a reasonable time frame. For example, the number of cells should be sufficient to bind to a cancer antigen, or detect, treat or prevent cancer in a period of from about 2 hours or longer, e.g., 12 to 24 or more hours, from the time of administration. In certain embodiments, the time period could be even longer. The number of cells will be determined by, e.g., the efficacy of the particular cells and the condition of the subject (e.g., human), as well as the body weight of the subject (e.g., human) to be treated.

Many assays for determining an administered number of cells from the inventive cell population expressing subject specific TCRs are known in the art. For purposes of the invention, an assay, which comprises comparing the extent to which target cells are lysed or one or more cytokines such as, e.g., IFN-gamma and IL-2 are secreted upon administration of a given number of such cells to a subject, could be used to determine a starting number to be administered to a mammal. The extent to which target cells are lysed, or cytokines such as, e.g., IFN-gamma and IL-2 are secreted, upon administration of a certain number of cells, can be assayed by methods known in the art. Secretion of cytokines such as, e.g., IL-2, may also provide an indication of the quality (e.g., phenotype and/or effectiveness) of a cell preparation.

The number of the cells administered from the inventive cell population expressing subject specific TCRs may also be determined by the existence, nature and extent of any adverse side effects that might accompany the administration of a particular cell population.

Subjects which may be treated are typically mammalian subjects - e.g. humans.

In one embodiment, the subject has been preselected on the basis of whether he/she expresses NRAS.Q61K or NRAS.Q61R mutation and has been identified as having a HLA-A*01:01 allotype.

Subject specific HLA alleles or HLA genotype of a subject may be determined by any method known in the art. In a particular embodiment, HLA genotypes are determined by any method described in International Patent Application number PCT/US2014/068746, published June 11, 2015 as WO2015085147. Briefly, the methods include determining polymorphic gene types that may comprise generating an alignment of reads extracted from a sequencing data set to a gene reference set comprising allele variants of the polymorphic gene, determining a first posterior probability or a posterior probability derived score for each allele variant in the alignment, identifying the allele variant with a maximum first posterior probability or posterior probability derived score as a first allele variant, identifying one or more overlapping reads that aligned with the first allele variant and one or more other allele variants, determining a second posterior probability or posterior probability derived score for the one or more other allele variants using a weighting factor, identifying a second allele variant by selecting the allele variant with a maximum second posterior probability or posterior probability derived score, the first and second allele variant defining the gene type for the polymorphic gene, and providing an output of the first and second allele variant.

The T cell populations disclosed herein are capable of being used in combination with at least one other therapeutic. Examples of therapeutics that can be used in conjunction with the T cells disclosed herein include, but are not limited to: immunomodulatory cytokines, including but not limited to, IL-2, IL-15, IL-7, IL-21, GM-CSF as well as any other cytokines that are capable of further enhancing immune responses; immunomodulatory antibodies, including but not limited to, anti-CTLA4, anti-CD40, anti-41BB, anti-OX40, anti-PD1 and anti-PDL1; and immunomodulatory drugs including, but not limited to, lenalidomide (Revlimid).

In addition, the T cell populations disclosed herein may be administered for cancer treatment in combination with chemotherapy in regimens that do not inhibit the immune system including, but not limited to, low dose cyclophosphamide and taxol. The vaccines may also be administered for cancer in combination with therapeutic antibodies including, but not limited to, anti-HER2/neu (Herceptin) and anti-CD20 (Rituxan).

The T cell populations can be administered for treatment of chronic infections in combination with drugs used to treat the particular type of infection including, but not limited to, anti-viral drugs, anti-retroviral drugs, anti-malarial drugs, etc.

In one embodiment, the agents of this aspect of the present invention are administered together with immune checkpoint inhibitors.

As used herein, the phrase "immune checkpoint inhibitor" refers to a compound capable of inhibiting the function of an immune checkpoint protein. Inhibition includes reduction of function and full blockade. In particular the immune checkpoint protein is a human immune checkpoint protein. Thus the immune checkpoint protein inhibitor preferably is an inhibitor of a human immune checkpoint protein. Immune checkpoint proteins are described in the art (see for instance Pardoll, 2012. Nature Rev. cancer 12: 252-264). The designation immune checkpoint includes the experimental demonstration of stimulation of an antigen-receptor triggered T lymphocyte response by inhibition of the immune checkpoint protein in vitro or in vivo, e.g. mice deficient in expression of the immune checkpoint protein demonstrate enhanced antigen-specific T lymphocyte responses or signs of autoimmunity (such as disclosed in Waterhouse et al., 1995. Science 270:985-988; Nishimura et al., 1999. Immunity 11:141-151). It may also include demonstration of inhibition of antigen-receptor triggered CD4+ or CD8+ T cell responses due to deliberate stimulation of the immune checkpoint protein in vitro or in vivo (e.g. Zhu et al., 2005. Nature Immunol. 6:1245-1252).

Preferred immune checkpoint protein inhibitors are antibodies that specifically recognize immune checkpoint proteins. A number of CTLA-4, PD1, PDL-1, PD-L2, LAG-3, BTLA, B7H3, B7H4, TIM3 and KIR inhibitors are known and in analogy of these known immune checkpoint protein inhibitors, alternative immune checkpoint inhibitors may be developed in the (near) future. For example ipilimumab is a fully human CTLA-4 blocking antibody presently marketed under the name Yervoy (Bristol-Myers Squibb). A second CTLA-4 inhibitor is tremelimumab (referenced in Ribas et al, 2013, J. Clin. Oncol. 31:616-22). Examples of PD-1 inhibitors include without limitation humanized antibodies blocking human PD-1 such as lambrolizumab (e.g. disclosed as hPD109A and its humanized derivatives h409A11, h409A16 and h409A17 in WO2008/156712; Hamid et al., N. Engl. J. Med. 369: 134-144 2013,), or pidilizumab (disclosed in Rosenblatt et al., 2011. J. Immunother. 34:409-18), as well as fully human antibodies such as nivolumab (previously known as MDX-1106 or BMS-936558, Topalian et al., 2012. N. Eng. J. Med. 366:2443-2454, disclosed in U.S. Pat. No. 8,008,449 B2). Other PD-1 inhibitors may include presentations of soluble PD-1 ligand including without limitation PD-L2 Fc fusion protein also known as B7-DC-Ig or AMP-244 (disclosed in Mkrtichyan M, et al. J Immunol. 189:2338-47 2012) and other PD-1 inhibitors presently under investigation and/or development for use in therapy. In addition, immune checkpoint inhibitors may include without limitation humanized or fully human antibodies blocking PD-L such as MEDI-4736 (disclosed in WO2011066389 A1), MPDL3280A (disclosed in U.S. Pat. No. 8,217,149 B2) and MIH1 (Affymetrix obtainable via eBioscience (16.5983.82)) and other PD-L1 inhibitors presently under investigation. According to this invention an immune checkpoint inhibitor is preferably selected from a CTLA-4, PD-1 or PD-L1 inhibitor, such as selected from the known CTLA-4, PD-1 or PD-L1 inhibitors mentioned above (ipilimumab, tremelimumab, labrolizumab, nivolumab, pidilizumab, AMP-244, MEDI-4736, MPDL3280A, MIH1). Known inhibitors of these immune checkpoint proteins may be used as such or analogues may be used, in particular chimerized, humanized or human forms of antibodies.

As used herein the term "about" refers to $\pm$ 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition. In one embodiment, the method is for substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the invention in a non limiting fashion.

## MATERIALS AND METHODS

### Data-driven analysis for recurrent-neoantigen candidate prioritization

*HLA/mutation co-occurrence analysis using patient data*

TCGA provisional cohorts data was used accumulating to a total of 8038 patients[37,61,62]. Previously published high-resolution HLA-I typing for 9176 TCGA patients were also obtained. Intersection of these two sources yields mutation- and HLA-annotated cohort of 6840 patients in total, 368 of whom are melanoma patients. Mutation and HLA data for 11033 patients pan-cancer, including 221 melanoma patients, was used and were processed as previously described. HLA-typed samples were annotated based on their RAS.Q61 status. Canonical transcript identifiers for NRAS (ENST00000369535), KRAS (ENST00000256078) and HRAS (ENST00000451590) Ensembl v101 on 31/08/2020, and their protein-coding sequence coordinates were obtained from Gencode v35 on 30/08/2020. The previously published MELA-AU dataset, consisting of 69 melanoma patients was obtained. For each of these three datasets, we computed pan-cancer and melanoma lists of all HLA-I alleles co-appearing with RAS.Q61 mutations. For each HLA/mutation combination we calculated its frequency in melanoma and pan-cancer. For the TCGA dataset, HLA-I frequencies were compared between the RAS.Q61 mutated and general populations (Fisher's exact test with FDR correction).

*NetMHCpan predictions*

We utilized *netMHCpan 4.0* to predict RAS.Q61-derived neopeptides[3]. Using NRAS' protein sequence, we extracted the 27 amino-acid long stretch flanking position 61. This sequence is conserved among RAS variants; therefore, it is representative of NRAS, KRAS and HRAS. Four mutated variants were constructed by substituting glutamine 61 with arginine, lysine, leucine or histidine. All five versions were wrapped into the FASTA format, and served as input to *netMHCpan,* along with the compiled list of HLA-alleles co-appearing with RAS.Q61 mutations in the analyzed patient-databases. The algorithm was set to scan for binding peptides of length 8-14 amino-acids, with peptides ranked at $0.5{\leq}\%$ Rank${\leq}2$ considered weak binders, and those with %Rank ${\leq}0.5$ as strong binders. The output was filtered to retain only peptides spanning the 61 position. A 'best %Rank' score was assigned to each HLA/mutation combination by enumerating over the predicted neopeptides.

### Tumor tissue, cell-lines, TIL and PBMC

Metastatic melanoma cell-lines 17T, 135T, 108T, and their cognate TIL products, were established. Established TIL were expanded via the Rapid Expansion Protocol (REP), as described previously[6].

The commercial melanoma cell-line SK-MEL-30 (ACC-151) was purchased from DSMZ. Additional tumor cell-lines: HuT78, Hs940T and Calu6, as well as HB95 (W6/32) and HB145 (IVA12) hybridoma cells, were purchased from ATCC. Healthy-donor leukocyte preparations, from blood-donations intended for research, were purchased from MDA (Israel's national blood services). PBMC were extracted from these using Ficoll-Paque PLUS (GE Healthcare) via density gradient centrifugation, cryopreserved in aliquots in FCS/10% DMSO and thawed as needed per experiments.

Hybridoma cells HB95 and HB145 were used to purify pan-HLA-I and pan-HLA-II antibodies for the preparation of the HLA affinity columns. All cell-lines were tested regularly and were found negative for mycoplasma contamination (EZ-PCR Mycoplasma Kit, Biological Industries).

Melanoma cell-lines were cultured in complete RPMI medium consisting of RPMI supplemented with 10% FCS, 2 mM L-glutamine, 2.5% HEPES, 100 IU/ml penicillin and 0.1 mg/ml streptomycin. B-LCLs were cultured in B-cell medium, consisting of complete RPMI medium supplemented with 1 mM sodium pyruvate. TIL and PBMC were cultured in T-cell medium consisting of RPMI supplemented with 10% heat inactivated human serum AB, 2 mM L-glutamine, 100 U/ml penicillin, 0.1 mg/ml Streptomycin and $5.5{\times}10^{-5}$M $\beta$-mercapto-ethanol. TIL were thawed and recovered in T-cell medium containing 6000 IU/ml IL-2 (Proleukin) for three days prior to use in downstream assays. PBMC were thawed and selected for T-cells in T-cell medium containing 300 IU/ml IL-2 and 50ng/ml OKT3 anti-CD3 antibody (#317304, Biolegend) seven days prior to experiments. PBMC were sub-cultured in T-cell medium supplemented with 300 IU/ml IL-2 at day 3-4 after thawing. T-cell medium was used for co-incubations in the caspase-3 killing and neoantigen induced expansion assays. Complete RPMI medium was used for overnight TIL/B-LCL and PBMC/B-LCL co-incubations.

## Structural modeling of HLA-A*01:01 in complex ILDTAGKEEY (SEQ ID NO: 1)

Structures of HLA-A*01:01 complexed with RAS.61-derived peptides were modelled using a crystal structure of HLA-A*01:01 in complex with an ALK tyrosine kinase receptor decapeptide (PDB: 6at9)[1]. The crystallographic bound peptide was manually mutated to yield the *ILDTAGKEEY* (SEQ ID NO: 1) and *ILDTAGQEEY* (SEQ ID NO: 35) peptides in complex with the HLA receptor. The HLA structure was truncated to the peptide-binding domain (chain A, residues 1 to 180). The resulting peptide-HLA structures were used as starting conformations for peptide docking and for molecule dynamics simulation.

Peptide docking was performed using the freely accessible web server interfaces FlexPepDock[2], ClusPro[3] and DINC[4]. In addition, molecular dynamics simulations were performed using GROMACS version 2018.3.5 with a GROMOS 54a7 united atom force field[6]. The complex was placed in a rhombic dodecahedral box with a minimum distance of 10 Å between the solute and box wall, and solvated by SPC water. The system's charge was neutralized by the addition of 5 Na+ counter ions. Steric clashes were removed by minimization, conducted using the steepest descent algorithm for a maximum of 5,000 steps. The system was equilibrated at constant volume and temperature (NVT ensemble) with all protein and peptide heavy atoms restrained for 100 ps at 10 K, followed by further equilibration without restraints for another 100 ps at 300 K. The system's pressure was equilibrated by simulation under constant atmospheric pressure (NPT ensemble) for 300 ps at 300 K. Positional restraints were applied to protein residues during all equilibration steps using the LINCS algorithm[7]. The final coordinates resulting from equilibration were used to commence five independent production runs for both (*ILDTAGKEEY* (SEQ ID NO: 1) and *ILDTAGQEEY*) (SEQ ID NO: 35) systems, each conducted for 500 ns in the NPT ensemble. The temperature was held constant at 300 K using the velocity rescaling thermostat8 coupled with a time constant of 0.1 ps, and the system's pressure was kept constant at 1 bar using the Parrinello-Rahman barostat[9] coupled with a time constant of 2 ps. A timestep of 2 fs was used to integrate the motions of the system. Long-range electrostatics were calculated using the Particle Mesh Ewald[10] method, while short-range cutoffs were set to 1.0 nm for both the vdW and Coulomb interactions. To focus on bound conformations, only conformations in which the RAS peptide's N- and C-termini were within 7 Å of the HLA B- and F-pockets, respectively, were analyzed. Distances were measured between the following atoms: HLA Tyr171 sidechain hydroxyl oxygen (OH) and peptide Ile1 backbone amide nitrogen (N), and between HLA Tyr123 sidechain phenolic carbon (CZ) and peptide Tyr10 sidechain gamma carbon (CG). Molecular structures were visualized using PyMOL version 1.3[11]. The conformations from docking and from bound simulation frames were clustered by HLA-peptide hydrogen bonding interactions. Hydrogen bonds were detected using the Wernet-Nilsson criteria[12] as implemented in MDTraj[13] version 1.9.2. Cluster centroids were identified as simulation frames for which the corresponding hydrogen bonding fingerprint possessed the lowest Manhattan/Cityblock distance to all cluster members.

## RAS.Q61 mutation status and HLA-typing of tumor tissue

For 17T and 108T, both RAS.Q61 status and HLA-typing were extracted from whole-exome sequencing; HLA-typing was determined using the *PolySolver* software[14]. For MM121224 and MM150414 HLA and mutation data were provided by its contributor. For 135T and snap-frozen tumors, RAS.Q61 status was informed by their contributors, while genomic DNA, extracted using QIAGEN's DNeasy Blood and Tissue kit (#69504), was utilized for HLA-typing (GenDx SBTexcellerator HLA-A kit #4100234 or the MX6-1 NGS typing kit, #7371464). Mutation and HLA data regarding cell-lines SK-MEL-30, MZ2-mel, HuT78, Hs940T and Calu6 were extracted from the "TRON cell line portal"[14,15]. HLA-typing of EBV-transformed B-cells was provided by the IHWG Cell and DNA Bank[16].

## cDNA sequencing

Total RNA was extracted from melanoma cell lines 17T, 135T, SK-MEL-30, MM121224, MZ2-mel, HuT78, Calu6 and Hs940T following the manufacturer's protocol using the RNeasy Mini Kit (#74104, QIAGEN), and eluted in 30 $\mu$l diethylpyrocarbonate (DEPC)-treated distilled $H_2O$. A total of 500 ng RNA was used for single-strand complementary DNA (cDNA) synthesis using the iScript Reverse Transcription Super mix for RT-qPCR kit (#1708841, Biorad) as per the manufacturer's protocol. NRAS and KRAS regions containing position 61 were amplified by PCR using the following primers:

NRAS forward: 5'-TTGGAGCAGGTGGTGTTGGG -3', (SEQ ID NO: 36);
NRAS reverse: 5'-GTATCAACTGTCCTTGTTGGC 3', (SEQ ID NO: 37);
KRAS forward: 5'-TAAACTTGTGGTAGTTGGAGCTGGT-3', (SEQ ID NO: 38); and
KRAS reverse: 5'-TTCTTTGCTGATTTTTTTCAA-3' (SEQ ID NO: 39).

2 $\mu$l of cDNA were taken for the PCR reaction, mixed with $2\times$ KAPA HIFI (#KM2605, KAPA Biosystems) to a final volume of 25 $\mu$l, using a standard PCR program with the following parameters: one cycle at 95 °C for 3 min; 35 cycles of 98 °C for 20 sec; annealing temperature of 58 °C for 30 sec; and 72 °C for 1 min. The PCR products were separated on a 1% agarose gel and then purified by Wizard SV Gel and PCR Clean-Up System (#A9281, Promega), followed by Sanger sequencing using a 3730 DNA Analyzer (ABI). The sequencing primers were the same as the PCR primers. The sequencing results were analyzed using the SnapGene software (Version 4.3.2).

**Establishment of double-transfectant 721.221**

DNA sequences coding for HLA-A*01:01 and 25-mer minigenes (RAS.Q61K and RAS.Q61R) were designed with flanking *XbaI* and *NotI* restriction sites at the 5' and 3' ends, respectively. The designs were purchased as synthetic dsDNA from either Twist bioscience or IDT (gBlock) following optimization for human expression via the respective companies' platforms. PCR amplified templates were restriction cloned using *XbaI/NotI* (NEB, #R0145L and # R0189L) into lentiviral vectors pCDH-CMV-MCS-EF1α-Neo (SBI, #CD514B-1) and pCDH-CMV-MCS-EF1α-GreenPuro (SBI, #CD513B-1). HLA and minigene inserts were ligated into the Neomycin and Puromycin vectors, respectively, using T4 DNA ligase (NEB, #M0202L) yielding pCDH-Neo-A*01:01, pCDH-Puro-GFP-mRAS.Q61K and pCDH-Puro-GFP-mRAS.Q61R. Sequence verified cloned plasmids were used to produce lentiviral particles via co-transfection with envelope and packaging plasmids PMD2.G and psPAX2 into HEK293T cells using Lipofectamine 2000 (Invitrogen, #11668027), per manufacturer's instructions. Harvested virus containing supernatant was filtered, aliquoted and stored at -80 °C. Cells of the HLA-I deficient human B-LCL 721.221 were stepwise infected, first to express HLA-A*01:01 and then with the minigene virus (RAS.Q61K or RAS.Q61R). 48-72 hours post infection selection was started either with 800 μg/ml neomycin (G418) or 1 μg/ml Puromycin. Infected cells were continuously propagated under selection. Productive over-expression of HLA-A*01:01 was validated approximately two weeks post infection via flow cytometry, using the monoclonal antibody W6/32. After validation of HLA expression, 721.221A*01:01 cells were infected and selected to induce minigene over-expression, as described above. 721.221A*01:01;RAS.Q61K and 721.221A*01:01;RAS.Q61R cells were grown in media containing both selection agents for approximately two weeks, and then propagated in antibiotics-devoid medium for additional two weeks before expansion for HLA-peptidomics.

**HLA-peptidomics**

*Production and purification of membrane HLA molecules*

Cell pellets consisting of 2x10⁸ cells each were collected and lysed on ice using a lysis buffer containing 0.25% sodium deoxycholate, 0.2 mM iodoacetamide, 1mM EDTA, 1:200 Protease Inhibitors Cocktail (Sigma-Aldrich, P8340), 1 mM PMSF and 1% octyl-β-D glucopyranoside in PBS. Samples were then incubated at 4 °C for 1 h. The lysates were cleared by centrifugation at 48,000 g for 60 min at 4 °C and then passed through a pre-clearing column containing Protein-A Sepharose beads.

HLA-I molecules were immunoaffinity purified from cleared lysate with the pan-HLA-I antibody (W6/32 antibody purified from HB95 hybridoma cells) covalently bound to Protein- A Sepharose beads (Thermo-Fisher Scientific, as reported previously)[17,18]. Affinity columns were washed first with 400 mM NaCl, 20 mM Tris HCl pH 8.0 and then with 20 mM Tris-HCl, pH 8.0. The HLA peptides and HLA molecules were then eluted with 1% trifluoracetic acid, followed by separation of the peptides from the proteins by binding the eluted fraction to Sep-Pak (Waters). Elution of the peptides was done with 28% acetonitrile in 0.1% trifluoracetic acid[17].

**Identification of eluted HLA peptides**

*Liquid chromatography:*

Cell lines 17T, SK-MEL-30 and MM121224: The HLA peptides were dried by vacuum centrifugation, solubilized with 0.1% formic acid, and resolved with a 7-40% acetonitrile gradient with 0.1% formic acid for 180 min and 0.15 μL/min on a capillary column pressurepacked with Reprosil C18-Aqua (Dr. Maisch, GmbH, Ammerbuch-Entringen, Germany) as previously described[19]. For cell lines 17T, SK-MEL-30 and MM121224, chromatography was performed with the UltiMate 3000 RSLCnano-capillary UHPLC system (Thermo Fisher Scientific), which was coupled by electrospray to tandem mass spectrometry on Q-Exactive-Plus (Thermo Fisher Scientific). HLA peptides were eluted over 2 h with a linear gradient from 5% to 28% acetonitrile with 0.1% formic acid at a flow rate of 0.15 μl/min.

Cell lines 721.221A*01:01; mRAS.Q61K, 721.221A*01:01; mRAS.Q61R ,135T, MZ2-MEL, HuT78, Hs940T and 4 tumor samples (Mela-183,Mela-49, MM-1369 and MM-1319): The HLA peptides were dried by vacuum centrifugation, solubilized with 97:3 water: acetonitrile 0.1% formic acid. Desalting of the samples was performed online using a reversed-phase Symmetry C18 trapping column (180 μm internal diameter, 20 mm length, 5 μm particle size; Waters). Peptides were resolved with a 5-28% acetonitrile gradient with 0.1% formic acid for 120 min using a T3 HSS nano-column (75 μm

internal diameter, 250 mm length, 1.8 μm particle size; Waters) at 0.35 μL/min. Chromatography was performed with the nanoAcquity (Waters), which was coupled by electrospray to tandem mass spectrometry on Q-Exactive-Plus (Thermo Fisher Scientific).

*Mass Spectrometry:* Cell lines 721.221A*01:01; mRAS.Q61K, 721.221A*01:01; mRAS.Q61R and 17T were run in discovery mode. Cell-lines: SK-MEL-30, MM121224 and 135T, as well as aliquots of the 721.221A*01:01; mRAS.Q61K ,721.221A*01:01; mNRAS.Q61R ,17T, MZ2-MEL, HuT78, Hs940T samples and 4 tumor samples (Mela-183,Mela-49, MM-1369 and MM-1319) were analyzed in an absolute targeted mass spectrometry, looking for *ILDTAGKEEY* (SEQ ID NO: 1) or *ILDTAGREEY* (SEQ ID NO: 34) specifically, and utilizing heavy-peptide spike-in, enabling also for peptide quantification. Synthetic heavy-isotope labeled *ILDTAGKEEY* (SEQ ID NO: 1), with heavy lysine (13C6;15N2) or *ILDTAGREEY* (SEQ ID NO: 34) with heavy arginine (13C6;15N4) incorporated, were purchased in >95% purity from *JPT.*

Discovery mode: Data was acquired using a data-dependent "top-10" method, fragmenting the peptides by higher-energy collisional dissociation. The full-scan MS spectra were acquired at a resolution of 70,000 at 200 m/z with a target value of $3 \times 10^6$ ions. Ions were accumulated to an automatic gain control (AGC) target value of $10^5$ with a maximum injection time of generally 100 msec. The peptide match option was set to *Preferred.* The normalized collision energy was set to 25% and the MS/MS resolution was 17,500 at 200 m/z. Fragmented m/z values were dynamically excluded from further selection for 20 sec. MS data were analyzed using MaxQuant (version 1.5.8.3)[20] with FDR 0.05. The peptide identifications were based on the human section of the UniProt database[21] (April 2017) and a customized reference database that contained the mutated sequences identified for 17T by WES.

SK-MEL-30, MM121224 absolute targeted mode: 0.1pmol heavy peptide was added to the peptidome sample injected into the mass-spectrometer. Analysis was then performed using the PRM method. An inclusion list was imported into the method for MS/MS acquisitions. The instrument switched between full MS and MS/MS acquisitions to fragment the ions in the inclusion list. Full-scan MS spectra were acquired at a resolution of 70,000, with a mass-tocharge ratio (m/z) of 350-1,400 AMU. Fragmented masses were accumulated to an AGC target value of $10^5$ with a maximum injection time of 400 msec and 1.8 m/z window. Analysis again utilized the MaxQuant software (version 1.5.8.3)[20] with the Andromeda search engine[22]. Peptide identifications were based on the human section of the UniProt database[21] (April 2017). and a customized reference database containing the *ILDTAGKEEY* (SEQ ID NO: 1) neo-antigen. The following parameters were used: precursor ion mass and fragment mass tolerance of 20 ppm, false discovery rate (FDR) of 0.05 for SK-MEL-30 and 0.3 for MM121224, and variable modification of oxidation (Met), acetylation (protein N-terminus) and heavy Lysine (13C6;15N2).

721.221A*01:01; mRAS.Q61K ,721.221A*01:01; mRAS.Q61R, 17T, 135T, MZ2-MEL, HuT78, Hs940T and 4 tumor samples (Mela-183,Mela-49, MM-1369 and MM-1319): absolute targeted mode:
The nanoUPLC was coupled online through a nanoESI emitter (10 μm tip; New Objective; Woburn, MA, USA) to a quadrupole orbitrap mass spectrometer (Q Exactive Plus, Thermo Scientific) using a FlexIon nanospray apparatus (Proxeon). Data was acquired in Parallel Reaction Monitoring (PRM) with one MS1 scan for every 10 PRM scans. MS1 scan range was set to 300-1800m/z, resolution of 70,000, AGC of 3e6 and maximum injection time was set to 120msec. The PRM channels were acquired at 35,000 resolution, maximum injection time of 200msec, AGC of 2e5, NCE of 27 and isolation of 1.7m/z.

Peptide quantification:

Raw PRM data was imported into Skyline[23]. Absolute quantification was obtained by summing extracted ion chromatograms of all fragment ions per peptide and exporting the ratio of total signal of the native peptide versus the heavy labeled internal standard that was spiked into the sample, multiplied by the amount of internal standard.

***Fluorescence-based in vitro killing assay***

In this assay, loss of fluorescent content is used to quantify target cell death24. GFP+ cells were established via lentiviral infection and antibiotic selection. Fluorescent target-cells were seeded at 70% confluence onto 48-well plates (1x105 and 5x104 cells/well for 17T and 135T, respectively) a day prior to the experiment and let adhere. Cognate TIL were added at effector to target ratios ranging from 0:1 to 4:1 and were co-incubated with the melanoma for 16 hours. A triplicate was included for each condition. After incubation, non-adherent TIL and dead target-cells were washed away with PBS. The fluorescence of remaining live target cells was quantified using Tecan-M200 plate reader. Fluorescence reading was focused 3 mm above the plate surface. The percentage of specific lysis was calculated as $100 \times (C-X)/C$, where C is fluorescence in the TIL-free condition and X is fluorescence in the presence of TILs.

**Peptide pulsing on B-LCLs**

Synthetic >95% pure peptides (*ILDTAGKEEY* (SEQ ID NO: 1), *ILDTAGREEY* (SEQ ID NO: 34), *ILDTAGQEEY*) (SEQ ID NO: 35) were purchased from GenScript and dissolved in DMSO. B-LCLs bearing HLA-A*01:01 were suspended in complete medium at $1 \times 10^6$ cells/ml, and incubated with the peptide of choice, at the desired concentration ($10^{-11}$-$10^{-5}$), for 2-4 h in a 37 °C, 5% $CO_2$, humidified incubator, in upright 15 ml conical tubes. DMSO volume was kept at 1% in all samples. For the no-peptide control, DMSO devoid of peptides was added. B-cells were washed in PBS three times before proceeding to the co-incubation with TILs.

**Analysis of T-cell reactivity by IFNγ release assay**

IFNy's release from TIL, as measured in an enzyme-linked immunosorbent assay (ELISA), was used to measure reactivity. TILs were co-cultured with either cognate melanoma or BLCLs at a 1:1 ratio ($10^5$-$2 \times 10^6$ cells) in U-bottom 96-well plates and incubated overnight in a 37 °C, 5% $CO_2$, humidified incubator. The soluble IFNγ secreted from TIL was quantified from the co-culture supernatant using Biolegend Human IFNγ ELISA MAX Deluxe (#430106), using Tecan-M200 plate reader. All experiments were conducted in biological triplicates.

*Flow cytometry analysis and fluorescence-activated cell sorting*

PBS 1% BSA 2 mM EDTA was utilized as staining and analysis buffer for flow cytometry experiments unless otherwise specified. Cells were passed through a 40 μm cell strainer (Corning, #431750) prior to sorting. Single-cell suspensions were analyzed in BD LSR II (BD Biosciences) or CytoFlex (Beckman Coulter) for flow cytometry. BD FACSAria III or BD FACSAria II Cell Sorters (BD Biosciences) were used for fluorescence-activated cell sorting. Cells were incubated with antibodies/tetramers for 30 minutes, at 4 °C in the dark and then washed. Tetramer and TCRβ or anti-CD8 staining were executed sequentially to avoid possible interference.

Tetramers were obtained from the NIH tetramer core facility, and were calibrated to minimize background staining: HLA-A*01:01/*ILDTAGKEEY* (SEQ ID NO: 1) (BV421-conjugated) was used at 1:8000-10000, A*01:01/*ILDTAGREEY* (SEQ ID NO: 34) (FITC-conjugated) was used at 1:4000. The following antibodies were purchased from Biolegend and utilized at 1:100 dilution: CD3 (PE/Cy7,#300316), CD4 (FITC, #317407), CD8 (APC #300911 or FITC #301006), 4-1BB (APC#309809), mouse TCRβ constant region (APC, #109211, Biolegend), CD19 (BV421,#302234). Staining with CellTrace far-red, anti-caspase3 and IFNγ capture antibodies was executed as described for the cleaved caspase3 killing and IFNγ secretion assays.

Size and granularity measures served to gate on viable singlets. Propidium iodine (Invitrogen #P3566) was used for live/dead staining, where specified. Sorting experiments gated on positive and negative sub-populations without overlap.

**IFNγ secretion assay**

To assess the percentage of A*01:01/*ILDTAGKEEY* (SEQ ID NO: 1) reactive TIL, we employed an IFNγ secretion assay (Miltenyi Biotec, #130-090-762), as per the manufacturer's instructions. In this assay, IFNγ specific antibody is attached to the cell surface and captures the secreted cytokine upon cellular release. IFNγ molecules bound to the cell surface are subsequently stained as artificial surface molecules and the cells analyzed by flow-cytometry. This approach is complementary to tetramer-staining, as neoantigen-specific cells are identified based induced reactivity rather than TCR recognition. IHW01161 cells, serving as APCs in these experiments, were pulsed with either no-peptide (DMSO) or 10 μg/ml mutant (*ILDTAGKEEY*) (SEQ ID NO: 1) or wild-type (*ILDTAGQEEY*) (SEQ ID NO: 35) peptide. TIL were co-incubated with APC for the duration of four hours. The no-peptide and wild-type conditions served as control scenarios and allowed to distinguish background from neoantigen-specific reactivity.

**Antigen induced TIL expansion assay**

IHW01161 cells, serving as APCs, were irradiated with 70 Gray, washed once with PBS, and then pulsed with 10 μg/ml of either mutant (*ILDTAGKEEY*) (SEQ ID NO: 1) or wild-type (*ILDTAGQEEY*) (SEQ ID NO: 35) peptide, as described above. 17TIL suspension of $2.5 \times 10^6$ cell/ml was prepared in T-cell medium and dispensed in 100 μl aliquots into wells of a 96-well round-bottom tissue culture plate. The mutant and wild-type pulsed APCs were resuspended each in T-cell medium at $2.5 \times 10^6$ cell/ml. Each well was added an additional 100 μl of either T-cell medium or pulsed APC suspension. Additional 1 μg/ml of mutant or wild-type peptide was added to the mutant/wild-type wells, respectively. Subsequently, cells were cultured for a total of ten days in 37 °C, 5% $CO_2$, humidified incubator. At day three of the incubation, half the medium was replaced with fresh T-cell medium containing 100 IU/ml IL-2, 50 ng/ml IL-7 (Peprotech, #200-07) and 50 ng/ml IL-15 (Peprotech, #200-15). At day seven of the incubation, half the medium was again replaced with fresh T-cell medium containing 200 IU/ml IL-2, 50 ng/ml IL-7 and 50 ng/ml IL-15. At day ten of incubation, cells were harvested, co-stained with anti-CD4 antibody and the A*01:01/ILDTAGKEEY (SEQ ID NO: 1) tetramer, and analyzed by flow cytometry.

**Bulk TCR sequencing**

We performed TCR library preparation on sorted TILs, as was previously described[25]. In short, RNA was extracted from TIL pools and treated with DNase (RNeasy Micro kit (QIAGEN), RQ1 RNase free DNase (Promega)). Reverse transcription was then performed using primers directed at the constant regions of the TCR $\alpha/\beta$ chains (SuperScript III, #18080044, Invitrogen). Single stranded oligonucleotides consisting of both a universal primer region and a unique molecular identifier (UMI) were ligated onto the 3' end of the TCR cDNA transcripts (T4 RNA ligase, #0204S, NEB). Over three consecutive PCR steps, the library was then adequately amplified and split into $\alpha$ and $\beta$ chain pools. Libraries were sequenced with 300 cycles on the NextSeq Illumina platform and were processed using an in-house pipeline.Mainly, reads were: (1) clustered according to UMI, for accurate frequency evaluation; (2) annotated for V and J germline gene segments according to the IMGT predetermined library with TRDV genes manually added[26]; and (3) determined for their CDR3 sequence at both the nucleotide and amino-acid level. The annotated output consisted of separate collections of $\alpha$ and $\beta$ chains, and was further filtered to exclude non-productive and singleton sequences. $0.5 \times 10^6$ cells were collected for each experimental condition. For the tetramer sorted experiment TIL were stained with HLA-A*01:01/ILDTAGKEEY (SEQ ID NO: 1) tetramer and anti-CD4 antibody and were sorted into: bulk CD4- and its tetramer+ and tetramer- subpopulations. All three populations were sequenced; the experiment was conducted in biological triplicates.

**Single-cell RNA and TCR sequencing of CD8+ TIL**

*TIL:melanoma co-incubation and tetramer sorting*

17TIL/17T and 135TIL/135T were plated in 1:1 ratio at $4 \times 10^6$ cells per well in a 24-well tissue culture plate. After co-incubation overnight, the cells were resuspended by pipetting, washed in PBS and then frozen in CryoStor CS10 (#07930, STEMCELL Technologies) at $7-10 \times 10^6$ cell/ml. Cells were thawed on the day of 10x library preparation, into Benzonase (#E8263, Millipore Sigma) supplemented complete medium, washed and then stained in complete medium. Cells were stained with the HLA-A*01:01/ILDTAGKEEY (SEQ ID NO: 1) tetramer, followed by staining for CD4 and CD8. Propidium iodine was used for live/dead staining.
The samples were sorted into complete medium, separating CD8+tetramer+ (CD8+CD4-tetramer+) from CD8+tetramer- (CD8+CD4-tetramer-) populations. Bulk CD8+ samples were also obtained by staining for CD4 and CD8 without tetramer staining and sorting for CD8+CD4-populations. Cells were forwarded to 10x library preparation immediately after sorting.

*10x library preparation and sequencing*

Samples were processed immediately after sorting, as described above, for single-cell library construction. Briefly, sorted cells were washed and resuspended with PBS containing 0.04% bovine serum albumin (BSA) and counted using trypan blue staining. Single cells were captured in droplets by loading onto the Chromium Controller with a targeted cell recovery of 10,000 cells per sample. Single-cell gene expression and TCR-enriched libraries were prepared using the Chromium Single Cell 5' V(D)J v1.1 Kit (10x Genomics) according to the manufacturer's protocol. Samples were sequenced on an Illumina NovaSeq with 26-bp read 1, 8-bp i7 index and 58-bp read 2 for gene expression libraries, and 150-bp paired end reads for TCR libraries.

*Data processing of scRNA and TCR-seq libraries*

Reads from 10x scRNA expression libraries were aligned to human genome assembly GRCh38 (hg38) and quantified using cellranger count (10x Genomics, version 3.1.0). The filtered feature-barcode matrices containing only cellular barcodes were used for further analysis. Single cell gene expression matrices were imported into R (version 3.6.1) and analyzed using Seurat (version 3.1.1). Cells with less than 4,000 or greater than 20,000 UMIs detected were filtered out. Additionally, cells with greater than 10% mitochondrial RNA reads were excluded from subsequent analyses.
Single cell TCR reads were aligned to human genome assembly GRCh38 (hg38) and assembled into reconstructed TCR consensus sequences using cellranger vdj (10x Genomics, version 3.1.0). Only productive TCR$\alpha$ and TCR$\beta$ sequences were considered for further analysis. Overall, TCR sequences were annotated for 95.2% of cells, with paired TCR$\alpha\beta$ sequences detected for 37,934 out of 41,542 cells (91.3%). Only cells with conventional paired TCR

chain combinations $\alpha\beta$ or $\alpha\alpha\beta$ were kept for downstream analyses (34,966 cells; 84.1%). Cells sharing the same CDR3$\alpha\beta$ amino acid sequences were defined as belonging to the same TCR clone.

### Identification of neoantigen-reactive clones:

TCR clonal frequencies were compared between the sorted tetramer+ and tetramerpopulations for each patient. Clones were considered as tetramer-specific if: (1) ≥100-fold higher clone frequency in the tetramer+ than in tetramer-populations, and (2) each TCR-chain is ≥100-fold enriched in the tetramer+ than in the tetramer- populations in at least one replicate of the bulk TCR sequencing experiments. 12 and 1 neoantigen-reactive clones were defined as tetramer-specific for 17TIL and 135TIL respectively, based on the above criteria. Tetramer specific clones consisting of at least five cells were retained for downstream neoantigen specific single-cell analyses. N17.5 (7 cells), N17.6 (2 cells) and N17.7 (one cell) were included based on TCR similarity criteria.

### Single cell data integration and clustering

In order to analyze the gene expression programs of neoantigen-reactive clones, the tetramer+ population (filtered for neoantigen-reactive clones) and bulk samples for both patients were integrated. Specifically, log normalization and variable feature selection based on variance stabilizing transformation was first performed on each sample individually. Then, anchors between datasets were identified using FindIntegrationAnchors, with the bulk samples as a reference and reciprocal PCA with 30 dimensions. The samples were then integrated using IntegrateData with 30 dimensions. The integrated expression matrix was then scaled and centered for each feature. Next, linear dimensional reduction was performed on the integrated scaled data using PCA, excluding TCR and cell cycle genes. For visualization, UMAP projections were calculated using the first 30 principal components. To identify clusters based on gene expression profiles, we performed shared nearest neighbor (SNN) modularity optimization clustering using a k parameter of 20 and a resolution of 0.5.

### TCR similarity analysis

For each of the three replicates of the bulk 17TIL TCR-sequencing experiment, we compared the tetramer+ repertoire to the bulk repertoire. We computed for each amino acid clonotype the ratio between the frequency in the tetramer+ repertoire to the frequency in the tetramerrepertoire. If the sequence was missing from the bulk repertoire, we estimated its frequency as $3\times10^{-5}$, which is slightly below the minimal frequency in the repertoires. We selected the sequences for which this ratio is above one (i.e., with higher frequency in the tetramer+ population). We concentrated on sequences that appear in this population for all three replicates of the experience. This procedure yielded ten $\alpha$ and eleven $\beta$ chains. Within this population, we looked for sequences of low Hamming distance from N135.1. We calculated for each $\beta$ sequence its sharing level in the Emerson data set[27], and for each $\alpha$ or $\beta$ sequence its probability of generation according to the standard human models delivered with OLGA[28], a model for estimating the probability of generation of a given CDR3 ($\alpha$ or $\beta$). Studying the prevalence of HLA-A*01:01 in the Emerson data set was based on the HLA annotations from DeWitt et al.[29].

### TCR overexpression in PBMC

This well documented technique was employed with slight modifications, as described below[30]. CDR3 nucleotide sequences were reconstructed with the appropriate V and J sequences as they appear in the IMGT reference[26], and were joined to mouse constant regions, as previously described[31]. Optimized mouse constant-region sequences were obtained. Each TCR chain was embedded between *XbaI*/*NotI* restriction sites. Sequences were ordered as dsDNA from *Twist bioscience,* following sequence optimization for human expression using the vendor's platform. TCR chains were restriction cloned into pGEM-4Z-EGFP-A64. Sequence-verified cloned plasmids were linearized with *SpeI* digestion, in-vitro transcribed using AmpliCap-Max T7 High Yield Message Maker Kit (#C-ACM04037, CellScript) and purified using RNAcleanup kit (#23600, Norgen Biotek). Formed mRNA was stored in 5$\mu$g aliquots at -80oC. PBMC were electroporated with mixed $\alpha$/$\beta$ mRNA, 10$\mu$g per 1x106 cells, at 400V/500$\mu$s, using an ElectroSquare Porator ECM 830 (BTX). Electroporated cells were rested for at least two hours prior to use in downstream experiments. TCR expression was validated via flowcytometry using mouse constant TCR$\beta$ staining.

### Peptide titration assay

HLA-A*01:01+ B-LCL were pulsed with synthetic peptide at concentrations ranging from $10^{-11}$M to $10^{-5}$M, as described above, and were subsequently co-incubated at 1:1 ratio with either TIL or electroporated lymphocytes for 16-20 hours in 96-well U-bottom plates, at 37°C 5% CO2 in a humidified incubator. Following co-incubation, plates were centrifuged at 300g. For 4-1BB readout, the cells pellets were stained for CD19, CD3, CD8 and 4-1BB and analyzed via flow-cytometry. The percentage of 4-1BB+ cells out of the CD8+ population was compared for the different conditions. For secreted IFNγ readout, the supernatant was collected and analyzed using the Human IFNγ ELISA MAX Deluxe (Biolegend, #430106), as above.

**Caspase-3 cleavage assay**

Intracellular staining of cleaved caspase-3 was utilized to measure cytotoxic T-cell-induced apoptosis of melanoma target cells as previously described[32]. Briefly, melanoma cells were stained with CellTrace Far Red (Invitrogen, #C34564), according to the manufacturer's protocol. $1 \times 10^5$ labeled melanoma cells were then co-cultured with lymphocytes at effector:target ratio of 3:1 in U-bottom 96-well plates for three hours at 37 °C, 5% CO2 in a humidified incubator. Following incubation, the cells were washed. fixed, permeabilized and labeled with an anti-cleaved caspase-3-PE antibody using the *Caspase 3 apoptosis PE kit* (BD, #550914) according to the manufacturer's protocol. Washed cells were analyzed by flow cytometry, to determine the percent of caspase-3+ cells out of CellTrace+ target cells. Donor derived lymphocytes electroporated with the TCR of interest were compared to negative controls of either lymphocytes electroporated with no mRNA or the melanoma cells alone. Experiment were conducted in biological triplicates.

*Statistical analysis*

Statistical analysis was executed using GraphPad Prism version 9.0.2 for MacOS, base R (version 3.5.0), and Real Statistics Resource Pack software for excel (Release 7.7.1, www(dot)real-statistics(dot)com). Comparison of HLA-allele frequencies between the pan-cancer, melanoma and RAS.Q61-mutated TCGA populations was conducted using Fisher's exact test with FDR correction. IFNg ELISA, 4-1BB based reactivity and cleaved caspase-3 assays were analyzed using one-way ANOVA, with Tukey's multiple comparisons test. Where multiple assays were conducted concurrently, and could therefore reliably compare, two-way ANOVA was utilized. Peptide-titration assays were analyzed via two-way ANOVA with Sidak's multiple comparisons test. Comparison of single-cell phenotypic proportions was conducted using Chi-squared test. Differences in activation scores between TCR clones were analyzed using Wilcoxon rank sum test with Bonferroni multiple hypothesis testing correction. Corrected P-values ≤0.05 were considered significant.

**REFERENCES FOR MATERIAL AND METHODS**

1. Toor JS, Rao AA, McShan AC, et al. A Recurrent Mutation in Anaplastic Lymphoma Kinase with Distinct Neoepitope Conformations. Front Immunol. 2018;9:99. doi: 1 0.3389/fimmu.20 18.00099

2. London N, Raveh B, Cohen E, Fathi G, Schueler-Furman O. Rosetta FlexPepDock web server--high resolution modeling of peptide-protein interactions. Nucleic Acids Res. 2011;39(Web Server issue):W249-53. doi:10.1093/nar/gkr431

3. Vajda S, Yueh C, Beglov D, et al. New Additions to the ClusPro Server Motivated by CAPRI. doi:10.1002/prot.25219

4. Antunes DA, Moll M, Devaurs D, Jackson KR, Lizée G, Kavraki LE. DINC 2.0: A New Protein-Peptide Docking Webserver Using an Incremental Approach. Cancer Res. 2017;77(21):e55-e57. doi:10.1158/0008-5472.CAN-17-0511

5. Páll S, Abraham MJ, Kutzner C, Hess B, Lindahl E. Tackling Exascale Software Challenges in Molecular Dynamics Simulations with GROMACS. In: Springer, Cham; 2015:3-27. doi:10.1007/978-3-319-15976-8_1

6. Schmid N, Eichenberger AP, Choutko A, et al. Definition and testing of the GROMOS force-field versions 54A7 and 54B7. Eur Biophys J. 2011;40(7):843-856. doi:10.1007/s00249-011-0700-9

7. Hess B, Bekker H, Berendsen HJC, Fraaije JGEM. LINCS: A linear constraint solver for molecular simulations. J Comput Chem. 1997;18(12):1463-1472. doi:10.1002/(SICI)1096-987X(199709)18:12<1463::AID-JCC4>3.0.CO;2-H

8. Bussi G, Donadio D, Parrinello M. Canonical sampling through velocity rescaling. J Chem Phys. 2007;126(1):014101. doi:10.1063/1.2408420

9. Parrinello M, Rahman A. Polymorphic transitions in single crystals: A new molecular dynamics method. J Appl Phys. 1981;52(12):7182-7190. doi:10.1063/1.328693

10. Essmann U, Perera L, Berkowitz ML, Darden T, Lee H, Pedersen LG. A smooth particle mesh Ewald method. J Chem Phys. 1995;103(19):8577-8593. doi:10.1063/1.470117

11. Schrodinger LLC. The PyMOL Molecular Graphics System, Version 1.8. 2015.

12. Wernet P, Nordlund D, Bergmann U, et al. The Structure of the First Coordination Shell in Liquid Water. Science (80- ). 2004;304(5673):995 LP - 999. doi:10.1126/science.1096205

13. McGibbon RT, Beauchamp KA, Harrigan MP, et al. MDTraj: A Modern Open Library for the Analysis of Molecular Dynamics Trajectories. Biophys J. 2015;109(8):1528-1532. doi:10.1016/J.BPJ.2015.08.01521

14. Shukla SA, Rooney MS, Rajasagi M, et al. Comprehensive analysis of cancer associated somatic mutations in class I HLA genes. Nat Biotechnol. 2015;33(11):1152-1158. doi:10.1038/nbt.3344

15. Scholtalbers J, Boegel S, Bukur T, et al. TCLP: an online cancer cell line catalogue integrating HLA type, predicted neo-epitopes, virus and gene expression. Genome Med. 2015;7(1):118. doi:10.1186/s13073-015-0240-5

16. IHWG FRED HUTCH Cell Lines & Genes. fredhutchdotorg/en/labs/clinical/projects/ihwg/cell-lines-genesdothtml. Accessed March 4, 2018.

17. Milner E, Gutter-Kapon L, Bassani-Strenberg M, Barnea E, Beer I, Admon A. The Effect of Proteasome Inhibition on the Generation of the Human Leukocyte Antigen (HLA) Peptidome. Mol Cell Proteomics. 2013;12(7):1853-1864. doi:10.1074/mcp.M112.026013

18. Bassani-Sternberg M, Barnea E, Beer I, Avivi I, Katz T, Admon A. Soluble plasma HLA peptidome as a potential source for cancer biomarkers. Proc Natl Acad Sci.2010;107(44):18769-18776. doi:10.1073/pnas.1008501107

19. Ishihama Y, Rappsilber J, Andersen JS, Mann M. Microcolumns with self-assembled particle frits for proteomics. J Chromatogr A. 2002;979(1-2):233-239. www(dot)ncbi(dot)nlm(dot)nih(dot)gov/pubmed/12498253. Accessed March 5, 2019.

20. Cox J, Mann M. MaxQuant enables high peptide identification rates, individualized p.p.b.-range mass accuracies and proteome-wide protein quantification. Nat Biotechnol. 2008;26(12):1367-1372. doi:10.1038/nbt.1511

21. UniProt: a worldwide hub of protein knowledge. Nucleic Acids Res.2019;47(D1):D506-D515. doi:10.1093/nar/gky1049

22. Cox J, Neuhauser N, Michalski A, Scheltema RA, Olsen J V., Mann M. Andromeda: A Peptide Search Engine Integrated into the MaxQuant Environment. J Proteome Res. 2011;10(4):1794-1805. doi:10.1021/pr101065j

23. Riaz N, Havel JJ, Makarov V, et al. Tumor and Microenvironment Evolution during Immunotherapy with Nivolumab. Cell. 2017;171(4):934-949.e16. doi:10.1016/J.CELL.2017.09.028

24. Steff A-M, Fortin M, Arguin C, Hugo P. Detection of a decrease in green fluorescent protein fluorescence for the monitoring of cell death: An assay amenable to high throughput screening technologies. Cytometry. 2001;45(4):237-243. doi:10.1002/1097-0320(20011201)45:4<237::AID-CYTO10024>3.0.CO;2-J

25. Bosselut R, Huseby E, Oakes T, et al. Quantitative Characterization of the t Cell Receptor Repertoire of Naive and Memory subsets Using an Integrated experimental and Computational Pipeline Which Is Robust, economical, and Versatile. Immunol. 2017;8:1267. doi:10.3389/fimmu.2017.01267

26. Greiff V, Miho E, Menzel U, Reddy ST. Bioinformatic and Statistical Analysis of Adaptive Immune Repertoires. Trends Immunol. 2015;36(11):738-749. doi:10.1016/j.it.2015.09.006

27. Emerson RO, DeWitt WS, Vignali M, et al. Immunosequencing identifies signatures of cytomegalovirus exposure history and HLA-mediated effects on the T cell repertoire. Nat Genet. 2017;49(5):659-665. doi:10.1038/ng.3822

28. Sethna Z, Elhanati Y, Callan CG, Walczak AM, Mora T. OLGA: fast computation of generation probabilities of B- and T-cell receptor amino acid sequences and motifs. Bioinformatics. 2019;35(17):2974-2981. doi:10.1093/bioinformatics/btz035

29. DeWitt WS, Smith A, Schoch G, Hansen JA, Matsen FA, Bradley P. Human T cell receptor occurrence patterns encode immune history, genetic background, and receptor specificity. Elife. 2018;7. doi:10.7554/eLife.3835822

30. Zhao Y, Zheng Z, Cohen CJ, et al. High-efficiency transfection of primary human and mouse T lymphocytes using RNA electroporation. Mol Ther. 2006;13(1):151-159. doi:10.1016/j.ymthe.2005.07.688

31. Cohen CJ, Zhao Y, Zheng Z, Rosenberg SA, Morgan RA. Enhanced antitumor activity of murine-human hybrid T-cell receptor (TCR) in human lymphocytes is associated with improved pairing and TCR/CD3 stability. Cancer Res. 2006;66(17):8878-8886. doi:10.1158/0008-5472.CAN-06-1450

32. He L, Hakimi J, Salha D, Miron I, Dunn P, Radvanyi L. A sensitive flow cytometrybased cytotoxic T-lymphocyte assay through detection of cleaved caspase 3 in target cells. J Immunol Methods. 2005;304(1-2):43-59. doi:10.1016/j.jim.2005.06.005.

## EXAMPLE 1

### *Selection of neoantigen candidates*

### Data-driven selection of neoantigen candidates

NRAS.Q61 is the second most highly mutated protein-position in melanoma, but not all of its derived neoantigens will be equally recurrent. To qualify as an 'interesting' hotspot-neoantigen, its *combined* HLA-allele/mutation frequency should be high in cancer patients. Here, we take a data-driven approach to neoantigen discovery by analyzing patients' genetic data to focus on high-potential candidates. Considering only those HLA-alleles that co-occur with RAS.Q61 mutations in the analyzed datasets, we utilize *NetMHCpan* to predict binding, 8-14 long, RAS.Q61-derived neopeptides. Using these data, we assigned each HLA/mutation combination with two scores: (1) its frequency in the cohort; and (2) its best binding-prediction score (i.e. best %Rank enumerated over the predicted binding neopeptides). We then applied stepwise filtration/sorting - setting a frequency threshold and then ordering the remaining candidates according to their estimated binding potential.

As the three RAS isoforms, i.e. NRAS, KRAS and HRAS, have an identical N-termini sequence, we included them all in our analyses, as were the four most prevalent RAS.Q61 amino-acid substitutions: arginine, lysine, leucine and histidine.

Using the above described scheme, we analyzed three melanoma cohorts: the TCGA, aggregating both mutation and HLA-I annotations for 363 melanoma cases, the Hartwig database, fostering 221 such patients, and the previously published MELA-AU dataset of 69 patients.

95 (26%), 60 (27%) and 15 (22%) individuals within these cohorts possess mutations at RAS.Q61, respectively. As expected, we found that the most frequent amino-acid substitutions at position 61 are arginine (46-60%) and lysine (35-47%); and NRAS is the most abundantly mutated RAS isoform at position 61 (96-100% of RAS.61 mutations).

Comparison of TCGA HLA-allele frequencies between RAS.Q61-mutant and -wild-type melanoma patients did not reveal significant prevalence skews (Fisher's exact test with FDR correction.

Plotting HLA/mutation combinations by their scores is demonstrative of how the most promising candidates were prioritized. Across the three datasets, HLA-A*01:01 stands out for contributing the two top candidates: HLA-A*01:01/RAS.Q61R and HLA-A*01:01/RAS.Q61K. We therefore selected HLA-A*01:01 for further analysis.

Consistent with its high abundance in the general population, 26-38% of patients within the analyzed cancer cohorts carry HLA-A*01:01[3]. Importantly, the allele's frequency does not diminish when restricted to the RAS.61-mutant population: 27-40% of RAS.Q61-mutant melanoma patients possess it. HLA-A*01:01 co-appears with RAS.Q61 mutations in 7-8.7% of melanoma. Specifically, 3.3-4.5% and 3-4.3% possess the combinations HLA-A*01:01/RAS.Q61R and HLA-A*01:01/RAS.Q61K combinations, respectively, in our analysis.

*NetMHCpan 4.0* predicts 32 RAS.Q61-derived neopeptides for A*01:01, including nine predicted strong binders. Importantly, the same 'canonical peptide', ILDTAGXEEY (SEQ ID NO: 40), is the top binding prediction for both RAS.Q61R and RAS.Q61K (predicted binding-affinities 202.4nM and 218.5nM, respectively), while its less prevalent variants (RAS.Q61L and RAS.Q61H) are also predicted to bind the A*01:01 allele (58.2nM and 101.8nM, respectively).

Of note, pan-cancer analysis recapitulates our melanoma-based candidate prioritization. Still frequent, RAS.Q61 mutations appear in 3.3-5.9% of the cases pan-cancer (226/6824 and 123/2091 individuals from the TCGA and Hartwig datasets, respectively). The relevance of an HLA-A*01:01/RAS.Q61 neoantigen will therefore not be restricted to melanoma.

**Direct identification of HLA-A\*01:01/RAS.Q61-derived neoantigens using mono-allelic over-expression system**

Unlike for RAS.Q61R, no neoantigen had been previously reported for the highly recurrent HLA-A*01:01/RAS.Q61K combination. Focusing on RAS.Q61K, we therefore set out to unbiasedly query the HLA-A*01:01-bound neoantigen landscape. To this end, we established a 721.221-based HLA-A*01:01 mono-allelic cell-line that co-overexpresses a 25-mer RAS.Q61K minigene, and subjected it to HLA-peptidomics. This overexpression setup has the advantages of availability and neoantigen-amplification, while still maintaining native antigen processing and presentation. Mass-spectrometry (MS) results were analyzed using *MaxQuant* and queried against the human proteome dataset (*Uniprot*), to which we manually added the minigene sequence. A total of 2403 peptides were detected for 721.221$^{A*01:01;mRAS.Q61K}$. While *NetMHCpan* predicts seven different HLA-A*01:01-binding neopeptides for RAS.Q61K, a single decapeptide, *ILDTAGKEEY* (SEQ ID NO: 1), was identified. This finding was further validated by comparing the identification spectra to that of a synthetic peptide. Interestingly, *ILDTAGKEEY* (SEQ ID NO: 1), and the closely related *ILDTAGREEY* (SEQ ID NO: 34) are the best scoring neopeptide predictions for RAS.Q61K and RAS.Q61R, respectively, in the context of HLA-A*01:01. We quantified neopeptide presentation in our overexpression system, as well as validated the presentation of RAS.Q61R-derived *ILDTAGREEY* (SEQ ID NO: 34), using high-sensitivity absolute targeted MS. *ILDTAGKEEY* (SEQ ID NO: 1) and *ILDTAGREEY* (SEQ ID NO: 34) were detected at 3.2850 and 38.2375 amol per 1x10$^6$ cells for 721.221$^{A*01:01;mRAS.Q61K}$ and 721.221$^{A*01:01;mRAS.Q61R}$, respectively.

**_ILDTAGKEEY_ (SEQ ID NO: 1) is robustly presented on HLA-A\*01:01/RAS.Q61K-expressing tumors**

To further validate our findings in the malignant context and under endogenous mutation/HLA expression, we subjected the 17T tumor-derived cell-line to HLA-peptidomics. As previous whole-exome efforts identified 17T melanoma as NRAS.Q61K mutant, and HLA-typing granted it HLA-A*01:01+, we employed MS in discovery-mode, to unbiasedly identify neoantigens, regardless of prediction or prior identifications. Analysis was executed as described above, with 17T mutated protein sequences, including the NRAS.Q61K variant, manually added to the human proteome as database. Indeed, among 2356 peptides, a single neopeptide was detected - decapeptide *ILDTAGKEEY* (SEQ ID NO: 1) (Figure 4).

To investigate the robustness of presentation, we curated a panel of four snap-frozen tumors and five additional tumor-derived cell-lines, all endogenously expressing the HLA-A*01:01/NRAS.Q61K combination. We included Hs940T, an HLA-A*01:01/NRAS.Q61R melanoma cell-line, in our analysis, to also test for endogenous presentation of *ILDTAGREEY* (SEQ ID NO: 34). All cell-lines were validated to express mutant NRAS transcripts.

HLA-peptidomics using high-sensitivity absolute targeted MS identified the *ILDTAGKEEY* (SEQ ID NO: 1) neopeptide in all NRAS.Q61K-mutant samples at quantities ranging from 0.1250 to 2.9138 amol per $1x10^6$ cells, assuming $1x10^8$ cells per 1mg of tumor tissue[41]. *ILDTAGREEY* (SEQ ID NO: 34) was identified as expected for Hs940T. Importantly, cell-line HuT78 from our panel is derived from a T-cell lymphoma, signifying the cross cancer-type relevance of the identified neoantigens.

We conclude that *ILDTAGKEEY* (SEQ ID NO: 1) is a robust, naturally processed, RAS.Q61K-derived neopeptide that is presented in the context of HLA allele A*01:01.

## EXAMPLE 2

### Identification of novel TCRs that binds neoantigens

### RESULTS

Mutant ILDTAGKEEY (SEQ ID NO: 1) was predicted to strongly bind HLA-A*01:01 as shown above and was previously validated to do so (WO2020/234875).

TCRs that target the HLA-A*01:01/ILDTAGKEEY (SEQ ID NO: 1) neoantigen have translational value; ACT with engineered T-cells utilizing them is expected to apply to 3% of melanoma cases, and to 2.9:1000 individuals pan-cancer. RNA-based TCR-sequencing was performed on tetramer-sorted TIL in order to identify relevant $\alpha$ and $\beta$ chains. For 17TIL and 135TIL each, three cell populations were individually sequenced: bulk CD4- and its A*01:01/ILDTAGKEEY (SEQ ID NO: 1)-tetramer+ and tetramer- subpopulations. The tetramer+ and tetramer- repertoires were contrasted in order to extract tetramer-enriched (i.e. tetramer-specific) chains, while sequencing of the bulk population allowed to place these chains in context. Sorting and sequencing experiments were performed in three biological replicates, and were highly consistent. While each sequencing experiment revealed hundreds to thousands of distinct, productive, TCR chains, both 17TIL and 135TIL clearly exhibited oligoclonal distributions. Restricting to amino-acid sequences that consist ≥1% of the transcripts, eleven $\beta$ (twelve $\alpha$ chains dominate 17TIL's CD4- repertoire, with cumulative coverage of 75.4% of the transcripts. Similarly, for 135TIL, six $\beta$ (seven $\alpha$) chains account for 92.2% of the CD4- repertoire. The tetramer+ and tetramer- subpopulations are likewise characterized by oligoclonal TCR distributions. Further focusing on TCR chains with

at least $100\text{-fold } \dfrac{\text{tetramer+}}{\text{tetramer}-}$ transcript frequency enrichment, four $\beta$ and five $\alpha$ chains emerge as neoantigen-specific for 17TIL. The cumulative frequencies for these four $\beta$ chains were 68.5%, 3% and 0.005% in the tetramer+, bulk CD4- and tetramer- populations, respectively. Similarly, cumulative frequencies for the five $\alpha$ chains were 68.9%, 3.2% and 0% in the tetramer+, bulk CD4- and tetramer- populations, respectively. For 135TIL, a single $\alpha\beta$ pair meets the above criteria (chains NA135.1 and NB135.1), with frequencies of 89.6% and 85% within tetramer+ 135TIL, respectively.

TCR-sequencing of sorted 4-1BB+ TIL, after overnight co-incubation with cognate melanoma, confirmed that indeed the chains of interest all take part in the melanoma-reactive repertoire, with two $\alpha$ and one $\beta$ of the chains significantly enriched in the 4-1BB+ subpopulation (binomial one-sided test, with Benjamini Hochberg correction).

### *Single-cell characterization of the TCR repertoire*

To further investigate these TCR repertoires, and to uncover tetramer-binding $\alpha\beta$ pairings, single-cell TCR-sequencing was performed. After overnight co-incubation of TIL and cognate melanoma (i.e. 17TIL with 17T, 135TIL with 135T), the samples were sorted into populations of interest; dual RNA- and TCR- single-cell sequencing using the 10x platform was then performed. Similar to the bulk sequencing experiment, the present inventors focused on three TIL populations: bulk CD8+ and its HLA-A*01:01/ILDTAGKEEY (SEQ ID NO: 1) -tetramer+ and tetramer-subpopulations.

$\dfrac{\text{tetramer+}}{\text{tetramer}-}$ frequency-enrichment was utilized to hone in on tetramer-specific clones.

As expected, the bulk and single-cell sequencing data are highly consistent. Candidate chain pairings, deduced from bulk TCR-sequencing, were confirmed by the single-cell data.

The present inventors focus on clones represented by at least 2 cells in the single-cell tetramer+ subpopulation. The leading tetramer-specific clones in 17TIL are hereon denoted N17.1-5, in decreasing order of frequency. The single dominant tetramer-specific clone for 135TIL is denoted N135.1. N17.2, N17.4, N17.5 and N135.1 are all αβ cells, while N17.3 is ααβ. Conventional analysis pipelines deemed N17.1 an αβ clone. However, as will be further elucidated below, this clone has in fact a second α chain, rearranged with the δ gene TRDV1. The respective TCR chains of the abovementioned clones will be designated as follows: *N{chain type: A/B}{TIL: 17/135}.{clone number}.{chain number[1]}*. For example, the β chain for clone N17.2 is written NB17.2, while the second α chain for clone N17.3 is NA17.3.2.

The sequences of the receptors are summarized in Table 1, herein below.

*Table 1*

| Clon e name | Neoanti gen- specific | cdr3s Amino acid: SEQ ID NO: | cdr3s nt SEQ ID NO: | TRA _v_ge ne | TRA _j_ge ne | TRA _c_ge ne | TRB _v_g ene | TRB _j_ge ne | TRB _c_ge ne |
|---|---|---|---|---|---|---|---|---|---|
| N13 5.1 | YES | TRA:2;TRB:3 | TRA:18; TRB: 19 | TRA V19 | TRA J39 | TRA C | TRB V6-1 | TRB J1-1 | TRB C1 |
| N17. 1.1 | NO | TRA:4;TRB:5 | TRA:20; TRB:21 | TRA V17 | TRA J49 | TRA C | TRB V27 | TRB J2-5 | TRB C2 |
| N17. 1.2 | YES | TRA:6;TRB:7 | TRA:22; TRB:23 | TRD V1 | TRA J27-1 | TRA C | TRB V27 | TRB J2-5 | TRB C2 |
| N17. 2 | YES | TRA:8;TRB:9 | TRA:24; TRB:25 | TRA V5 | TRA J9 | TRA C | TRB V6-6 | TRB J2-7 | TRB C2 |
| N17. 3.1 | NO | TRA:10;TRB: 11 | TRA:26; TRB:27 | TRA V16 | TRA J52 | TRA C | TRB V6-1 | TRB J1-1 | TRB C1 |
| N17. 3.2 | YES | TRA:12;TRB: 13 | TRA:28; TRB:29 | TRA V19 | TRA J17 | TRA C | TRB V6-1 | TRB J1-1 | TRB C1 |
| N17. 4 | | TRA:14;TRB: 15 | TRA:30; TRB:31 | TRA V13-2 | TRA J6 | TRA C | TRB V12-4 | TRB J1-1 | TRB C1 |
| N17. 5 | YES | TRA:16;TRB 17 | TRA:32; TRB:33 | TRA V19 | TRA J39 | TRA C | TRB V6-6 | TRB J1-1 | TRB C1 |

***Validation and characterization of the neoantigen-binding TCR repertoire*** Having identified multiple tetramer-specific T-cell clones, the present inventors aimed to validate their individual sensitivity and specificity towards the HLA-A*01:01/ILDTAGKEEY (SEQ ID NO: 1) neoantigen.

To this end, they overexpressed α/β combinations of interest in healthy-donor peripheral T-cells. As depicted in Figure 1A, tetramer-binding experiments confirmed neoantigen-binding for N17.2, N17.5 and N135.1, and discerned the relevant αβ combination for ααβ clone N17.3 (NA17.3.2/NB17.3). Surprisingly, the αβ pair pertaining to the most prominent of 17TIL's tetramer-specific clones, N17.1, did not validate, albeit efficient expression (Figure 1A). Clonal expression of δ-gene TRDV1 in this clone, as observed in single-cell RNA-sequencing, has led us to reinspect the TCR-sequencing data and to uncover a second α chain for N17.1, NA17.1.2, resulting from an uncommon event of TRDV1/TRAJ27 recombination[46]. NA17.1.2 has been identified in frequency equivalent to that of NA17.1.1 in bulk TCR-sequencing. No other TRDV genes were implicated in α-chain rearrangements. As expected, the NA17.1.2/NB17.1 chain combination validated on tetramer-staining (Figure 1A).

The present inventors proceeded to co-incubate the different TCR-engineered T-cells with HLA-A*01:01+ B-LCL pulsed with either the ILDTAGKEEY (SEQ ID NO: 1) neopeptide or its wild-type counterpart at different concentrations. All five tested TCRs proved to be neoantigen-specific, as evidenced by differential reactivity towards the mutated peptide at supraphysiologic pulsed-peptide concentrations (Figure 1B). Peptide titration experiments further elucidated that these TCRs potently recognize the ILDTAGKEEY (SEQ ID NO: 1) neopeptide at concentrations ranging from 0.01nM to 10nM (Figure 1C). Interestingly, a trend towards inverse relationship between TCR frequency and sensitivity within 17TIL was observed. To further gauge the TCRs' reactivity and cytotoxic-capacity towards endogenously-expressed neoantigen, the present inventors co-incubated TCR-engineered T-cells with HLA-A*01:01+NRAS.Q61K+ tumor cell-lines. Testing the two most potent TCRs, N17.1.2 and N17.2, in a reactivity assay against a panel of tumor cell-lines recapitulated the immunopeptidomics results (Figure 2A). Moreover, significant 4-1BB upregulation was observed upon co-incubation with the HLA-A*01:01+KRAS.Q61K+ lung adenocarcinoma cell-line Calu6, attesting to the cross-isoform, cross-cancer-type relevance of the HLA-A*01:01/ILDTAGKEEY (SEQ ID NO: 1) neoantigen, as predicted. All five tested TCRs possess cytotoxic capacity towards neoantigen-expressing melanoma, as demonstrated in a cleaved Caspase3 assay (Figure 2B).

Since the majority of tetramer-binding 17TIL cross-binds also to the HLA-A*01:01/ILDTAGREEY (SEQ ID NO: 34)-tetramer, it was speculated that it is N17.1.2 that possess both reactivities. Remarkably, indeed, N17.1.2 proved to recognize the ILDTAGREEY (SEQ ID NO: 34) neopeptide at concentration as low as 1nM and to induce 4-1BB upregulation upon co-incubation with HLA-A*01:01+/NRAS.Q61R+ cell-lines (Figures 1C and 2A). The remaining four TCRs were confirmed to not recognize the ILDTAGREEY (SEQ ID NO: 34) neopeptide.

***Intra- and inter-patient sequence convergence of neoantigen-specific T-cell receptors***

The present inventors investigated whether some of the uncovered $\alpha/\beta$ chains (as presented in Table 1) are interchangeable. Depicted in Figures 3A-F, NH1 (NA135.1/NB17.5) is a hypothetical clone which maximizes $P_{gen}$ for both $\alpha$ and $\beta$ within the similarity cluster. NH1 was tested for neoantigen-reactivity and was validated both with tetramer-staining and neoantigen-specific IFN$\gamma$ release (Figures 3A-F). The reverse combination, NA17.5/NB135.1, validated as well.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

It is the intent of the applicant(s) that all publications, patents and patent applications referred to in this specification are to be incorporated in their entirety by reference into the specification, as if each individual publication. patent or patent application was specifically and individually noted when referenced that it is to be incorporated herein by reference. In addition, citation or identification of any reference in this application shall not be construed as an admission that such reference is available as prior art to the present invention. To the extent that section headings are used, they should not be construed as necessarily limiting. In addition, any priority document(s) of this application is/are hereby incorporated herein by reference in its/their entirety.

## Claims

1. A population of T cells for use in treating cancer of a subject, wherein an alpha chain of a T cell receptor (TCR) of at least 10 % of said T cells of said population has a CDR3 amino acid sequence as set forth in SEQ ID NO: 6, and a beta chain of said TCR has a CDR3 amino acid sequence as set forth in SEQ ID NO: 7.

2. The population of T cells for use according to claim 1, wherein the subject is HLA-A*01:01/NRAS.Q61K or HLA-A*01:01/NRAS.Q61R.

3. The population of T cells for use according to claims 1 or 2, wherein said T cells are autologous to the subject.

4. The population of T cells for use according to claims 1 or 2, wherein said T cells are non-autologous to the subject.

5. The population of T cells for use according to any one of claims 1-4, wherein said T cells are genetically modified to express said T cell receptor.

6. The population of T cells for use according to any one of claims 1-5, wherein said T cells comprise CD8+ T cells.

7. The population of T cells for use according to any one of claims 1-6, wherein said cancer is selected from the group consisting of melanoma, colon cancer, breast cancer, thyroid cancer, stomach cancer, colorectal cancer, leukemia cancer, bladder cancer, lung cancer, ovarian cancer, breast cancer and prostate cancer.

8. The population of T cells for use according to any one of claims 1-7, wherein said cancer is melanoma.

9. The population of T cells for use according to any one of claims 1-8, wherein the subject is being treated with a checkpoint inhibitor.

10. An isolated population of T cells genetically modified to express a T cell receptor (TCR), wherein an alpha chain of said TCR has a CDR3 amino acid sequence as set forth in SEQ ID NO: 6, and a beta chain of said TCR has a CDR3 amino acid sequence as set forth in SEQ ID NO: 7.

11. An isolated population of T cells, wherein an alpha chain of a TCR of at least 10 % of the T cells of the population has a CDR3 amino acid sequence as set forth in SEQ ID NO: 6, and a beta chain of said TCR has a CDR3 amino acid sequence as set forth in SEQ ID NO: 7.

12. The isolated population of T cells of claims 10 or 11, being CD8+ T cells.

**Patentansprüche**

1. Eine Population von T-Zellen zur Verwendung bei der Behandlung einer Krebserkrankung eines Versuchsobjekts, wobei eine alpha-Kette eines T-Zell-Rezeptors (TCR) von mindestens 10% der T-Zellen dieser Population eine CDR3-Aminosäuresequenz wie in SEQ ID NO: 6 dargelegt aufweist, und eine beta-Kette des TCR eine CDR3-Aminosäuresequenz wie in SEQ ID NO: 7 dargelegt aufweist.

2. Population von T-Zellen zur Verwendung nach Anspruch 1, wobei das Versuchsobjekt HLA-A*01:01/NRAS.Q61K oder HLA-A*01:01/NRAS.Q61R ist.

3. Population von T-Zellen zur Verwendung nach Anspruch 1 oder 2, wobei die T-Zellen dem Versuchsobjekt körpereigen sind.

4. Population von T-Zellen zur Verwendung nach Anspruch 1 oder 2, wobei die T-Zellen dem Versuchsobjekt nicht körpereigen sind.

5. Population von T-Zellen zur Verwendung nach einem der Ansprüche 1 bis 4, wobei die T-Zellen gentechnisch verändert sind, um den T-Zell-Rezeptor zu exprimieren.

6. Population von T-Zellen zur Verwendung nach einem der Ansprüche 1 bis 5, wobei die T-Zellen CD8+-T-Zellen umfassen.

7. Population von T-Zellen zur Verwendung nach einem der Ansprüche 1 bis 6, wobei die Krebserkrankung ausgewählt wird aus der Gruppe bestehend aus Melanomkrebs, Darmkrebs, Brustkrebs, Schilddrüsenkrebs, Magenkrebs, Dickdarmkrebs, Blutkrebs, Blasenkrebs, Lungenkrebs, Eierstockkrebs, Brustkrebs und Prostatakrebs.

8. Population von T-Zellen zur Verwendung nach einem der Ansprüche 1 bis 7, wobei die Krebserkrankung Melanomkrebs ist.

9. Population von T-Zellen zur Verwendung nach einem der Ansprüche 1 bis 8, wobei das Versuchsobjekt mit einem Checkpoint-Inhibitor behandelt wird.

10. Isolierte Population von T-Zellen, die gentechnisch verändert ist, um einen T-Zell-Rezeptor (TCR) zu exprimieren, wobei eine alpha-Kette des TCR eine CDR3-Aminosäuresequenz wie in SEQ ID NO: 6 dargelegt aufweist, und eine beta-Kette des TCR eine CDR3-Aminosäuresequenz wie in SEQ ID NO: 7 dargelegt aufweist.

11. Isolierte Population von T-Zellen, wobei eine alpha-Kette eines TCR von mindestens 10% der T-Zellen der Population eine CDR3-Aminosäuresequenz wie in SEQ ID NO: 6 dargelegt aufweist, und eine beta-Kette des TCR eine CDR3-Aminosäuresequenz wie in SEQ ID NO: 7 dargelegt aufweist.

12. Isolierte Population von T-Zellen nach Anspruch 10 oder 11, die aus CD8+-Zellen besteht.

**Revendications**

1. Population de lymphocytes T pour une utilisation dans le traitement d'un cancer d'un sujet, dans laquelle une chaîne alpha d'un récepteur de lymphocyte T (TCR) d'au moins 10 % desdits lymphocytes T de ladite population présente une séquence d'acides aminés de CDR3 telle que représentée par SEQ ID NO : 6, et une chaîne bêta dudit TCR présente une séquence d'acides aminés de CDR3 telle que représentée par SEQ ID NO : 7.

2. Population de lymphocytes T pour une utilisation selon la revendication 1, dans laquelle le sujet est HLA-A*01:01/NRAS.Q61K ou HLA-A*01:01/NRAS.Q61R.

3. Population de lymphocytes T pour une utilisation selon les revendications 1 ou 2, dans laquelle lesdits lymphocytes T sont autologues par rapport au sujet.

4. Population de lymphocytes T pour une utilisation selon les revendications 1 ou 2, dans laquelle lesdits lymphocytes T sont non autologues par rapport au sujet.

5. Population de lymphocytes T pour une utilisation selon l'une quelconque des revendications 1 à 4, dans laquelle lesdits lymphocytes T sont génétiquement modifiés pour exprimer ledit récepteur de lymphocyte T.

6. Population de lymphocytes T pour une utilisation selon l'une quelconque des revendications 1 à 5, dans laquelle lesdits lymphocytes T comportent des lymphocytes T CD8+.

7. Population de lymphocytes T pour une utilisation selon l'une quelconque des revendications 1 à 6, dans laquelle ledit cancer est sélectionné dans le groupe consistant en un mélanome, un cancer du côlon, un cancer du sein, un cancer de la thyroïde, un cancer de l'estomac, un cancer colorectal, une leucémie, un cancer de la vessie, un cancer du poumon, un cancer de l'ovaire, un cancer du sein et un cancer de la prostate.

8. Population de lymphocytes T pour une utilisation selon l'une quelconque des revendications 1 à 7, dans laquelle ledit cancer est un mélanome.

9. Population de lymphocytes T pour une utilisation selon l'une quelconque des revendications 1 à 8, dans laquelle le sujet est traité avec un inhibiteur de point de contrôle.

10. Population isolée de lymphocytes T génétiquement modifiés pour exprimer un récepteur de lymphocyte T (TCR), dans laquelle une chaîne alpha dudit TCR présente une séquence d'acides aminés de CDR3 telle que représentée par SEQ ID NO : 6, et une chaîne bêta dudit TCR présente une séquence d'acides aminés de CDR3 telle que représentée par SEQ ID NO : 7.

11. Population isolée de lymphocytes T, dans laquelle une chaîne alpha d'un TCR d'au moins 10 % des lymphocytes T de la population présente une séquence d'acides aminés de CDR3 telle que représentée par SEQ ID NO : 6, et une chaîne bêta dudit TCR présente une séquence d'acides aminés de CDR3 telle que représentée par SEQ ID NO : 7.

12. Population isolée de lymphocytes T selon les revendications 10 ou 11, qui sont des lymphocytes T CD8+.

FIG. 1A

FIG. 1B

FIG. 1C

FIG. 2A

FIG. 2B

FIG. 3A

FIG. 3B

FIG. 3C

FIG. 3D

FIG. 3E

FIG. 3F

FIG. 4

EP 4 329 780 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63223114
- IL 282814
- WO 2020234875 A
- WO 2020037302 A
- WO 2019075112 A
- WO 2020180648 A
- WO 2019226939 A
- WO 2019168984 A
- WO 2019226941 A
- WO 2019133853 A
- WO 2019036688 A
- WO 2018227030 A

- WO 2019050994 A
- WO 2018208856 A
- WO 2018195357 A
- WO 2018098362 A
- WO 2019104203 A
- WO 2017106638 A
- US 2014068746 W
- WO 2015085147 A
- WO 2008156712 A
- US 8008449 B2
- WO 2011066389 A1
- US 8217149 B2

**Non-patent literature cited in the description**

- **REUSCHENBACH et al.** *Cancer Immunol. Immunother.*, 2009, vol. 58, 1535-1544
- **PARMIANI et al.** *J. Nat. Cancer Inst.*, 2002, vol. 94, 805-818
- **ZAROUR et al.** *Cancer Medicine.*, 2003
- **BRIGHT et al.** *Hum. Vaccin. Immunother.*, 2014, vol. 10, 3297-3305
- **WURZ et al.** *Ther. Adv. Med. Oncol.*, 2016, vol. 8, 4-31
- **CRISCITIELLO**. *Breast Care*, 2012, vol. 7, 262-266
- **CHESTER et al.** *J. Immunother. Cancer*, 2015, vol. 3, 7
- **LI et al.** *Mol. Med. Report*, 2008, vol. 1, 589-594
- **LIU et al.** *J. Hematol. Oncol.*, 2010, vol. 3, 7
- **BERTINO et al.** *Biomed. Res. Int.*, 2015, 731469
- **SURI et al.** *World J. Gastrointest. Oncol.*, 2015, vol. 7, 492-502
- **NG, P C et al.** *PLoS Gen.*, 2008, vol. 4 (8), 1-15
- *J Immunol Methods*, 2011, vol. 374, 1-4
- **KALAORA et al.** *Oncotarget.*, 02 February 2016, vol. 7 (5), 5110-5117
- **RININSLAND et al.** *J Immunol Methods*, 2000, vol. 240 (1-2), 143-155
- **MURALI-KRISHNA et al.** *Adv Exp Med Biol.*, 1998, vol. 452, 123-142
- **WALKER et al.** *Nature*, 1987, vol. 328, 345-348
- **SCHEIBENBOGEN et al.** *J Immunol Methods*, 2000, vol. 244 (1-2), 81-89

- **BESSER et al.** *Clin. Cancer Res*, 2010, vol. 16 (9), 2646-55
- **DUDLEY et al.** *Science*, 2002, vol. 298 (5594), 850-4
- **DUDLEY et al.** *Journal of Clinical Oncology*, 2005, vol. 23 (10), 2346-57
- **JOHNSON et al.** *Blood*, 2009, vol. 114 (3), 535-46
- **MORGAN et al.** *Science*, 2006, vol. 314 (5796), 126-9
- **KALOS et al.** *Science Translational Medicine*, 2011, vol. 3 (95), 95-73
- **PING et al.** *Protein Cell.*, March 2018, vol. 9 (3), 254-266
- **GREEN ; SAMBROOK**. Molecular Cloning: A Laboratory Manual. Cold Spring Harbor Press, 2012
- **PARDOLL**. *Nature Rev. cancer*, 2012, vol. 12, 252-264
- **WATERHOUSE et al.** *Science*, 1995, vol. 270, 985-988
- **NISHIMURA et al.** *Immunity*, 1999, vol. 11, 141-151
- **ZHU et al.** *Nature Immunol.*, 2005, vol. 6, 1245-1252
- **RIBAS et al.** *J. Clin. Oncol.*, 2013, vol. 31, 616-22
- **HAMID et al.** *N. Engl. J. Med.*, 2013, vol. 369, 134-144
- **ROSENBLATT et al.** *J. Immunother.*, 2011, vol. 34, 409-18
- **TOPALIAN et al.** *N. Eng. J. Med.*, 2012, vol. 366, 2443-2454

- **MKRTICHYAN M et al.** *J Immunol.*, 2012, vol. 189, 2338-47
- **TOOR JS** ; **RAO AA** ; **MCSHAN AC et al.** A Recurrent Mutation in Anaplastic Lymphoma Kinase with Distinct Neoepitope Conformations.. *Front Immunol.*, 2018, vol. 9, 99
- **LONDON N** ; **RAVEH B** ; **COHEN E** ; **FATHI G** ; **SCHUELER-FURMAN O**. Rosetta FlexPepDock web server--high resolution modeling of peptide-protein interactions.. *Nucleic Acids Res.*, 2011, vol. 39, W249-53
- **VAJDA S** ; **YUEH C** ; **BEGLOV D et al.** *New Additions to the ClusPro Server Motivated by CAPRI*
- **ANTUNES DA** ; **MOLL M** ; **DEVAURS D** ; **JACKSON KR** ; **LIZÉE G** ; **KAVRAKI LE**. DINC 2.0: A New Protein-Peptide Docking Webserver Using an Incremental Approach.. *Cancer Res.*, 2017, vol. 77 (21), e55-e57
- **PÁLL S** ; **ABRAHAM MJ** ; **KUTZNER C** ; **HESS B** ; **LINDAHL E**. Tackling Exascale Software Challenges in Molecular Dynamics Simulations with GROMACS. Springer, 2015, 3-27
- **SCHMID N** ; **EICHENBERGER AP** ; **CHOUTKO A et al.** Definition and testing of the GROMOS force-field versions 54A7 and 54B7. *Eur Biophys J.*, 2011, vol. 40 (7), 843-856
- **HESS B** ; **BEKKER H** ; **BERENDSEN HJC** ; **FRAAIJE JGEM**. LINCS: A linear constraint solver for molecular simulations.. *J Comput Chem.*, 1997, vol. 18 (12), 1463-1472
- **BUSSI G** ; **DONADIO D** ; **PARRINELLO M**. Canonical sampling through velocity rescaling.. *J Chem Phys.*, 2007, vol. 126 (1), 014101
- **PARRINELLO M** ; **RAHMAN A**. Polymorphic transitions in single crystals: A new molecular dynamics method.. *J Appl Phys.*, 1981, vol. 52 (12), 7182-7190
- **ESSMANN U** ; **PERERA L** ; **BERKOWITZ ML** ; **DARDEN T** ; **LEE H** ; **PEDERSEN LG**. A smooth particle mesh Ewald method.. *J Chem Phys.*, 1995, vol. 103 (19), 8577-8593
- *The PyMOL Molecular Graphics System, Version 1.8.*, 2015
- **WERNET P** ; **NORDLUND D** ; **BERGMANN U et al.** The Structure of the First Coordination Shell in Liquid Water.. *Science*, 2004, vol. 304 (5673), 995 LP-999
- **MCGIBBON RT** ; **BEAUCHAMP KA** ; **HARRIGAN M et al.** MDTraj: A Modern Open Library for the Analysis of Molecular Dynamics Trajectories.. *Biophys J.*, 2015, vol. 109 (8), 1528-1532
- **SHUKLA SA** ; **ROONEY MS** ; **RAJASAGI M et al.** Comprehensive analysis of cancer associated somatic mutations in class I HLA genes.. *Nat Biotechnol.*, 2015, vol. 33 (11), 1152-1158
- **SCHOLTALBERS J** ; **BOEGEL S** ; **BUKUR T et al.** TCLP: an online cancer cell line catalogue integrating HLA type, predicted neo-epitopes, virus and gene expression.. *Genome Med.*, 2015, vol. 7 (1), 118
- **IHWG FRED HUTCH**. *Cell Lines & Genes.*, 04 March 2018
- **MILNER E** ; **GUTTER-KAPON L** ; **BASSANI-STRENBERG M** ; **BARNEA E** ; **BEER I** ; **ADMON A**. The Effect of Proteasome Inhibition on the Generation of the Human Leukocyte Antigen (HLA) Peptidome.. *Mol Cell Proteomics.*, 2013, vol. 12 (7), 1853-1864
- **BASSANI-STERNBERG M** ; **BARNEA E** ; **BEER I** ; **AVIVI I** ; **KATZ T** ; **ADMON A**. Soluble plasma HLA peptidome as a potential source for cancer biomarkers.. *Proc Natl Acad Sci.*, 2010, vol. 107 (44), 18769-18776
- **ISHIHAMA Y** ; **RAPPSILBER J** ; **ANDERSEN JS** ; **MANN M**. Microcolumns with self-assembled particle frits for proteomics.. *J Chromatogr A.*, 05 March 2019, vol. 979 (1-2), 233-239, www(dot)ncbi(dot)nlm(dot)nih(dot)gov/pubmed/12498253
- **COX J** ; **MANN M**. MaxQuant enables high peptide identification rates, individualized p.p.b.-range mass accuracies and proteome-wide protein quantification.. *Nat Biotechnol.*, 2008, vol. 26 (12), 1367-1372
- UniProt: a worldwide hub of protein knowledge.. *Nucleic Acids Res.*, 2019, vol. 47 (D1), D506-D515
- **COX J** ; **NEUHAUSER N** ; **MICHALSKI A** ; **SCHELTEMA RA** ; **OLSEN J V.** ; **MANN M**. Andromeda: A Peptide Search Engine Integrated into the MaxQuant Environment.. *J Proteome Res.*, 2011, vol. 10 (4), 1794-1805
- **RIAZ N** ; **HAVEL JJ** ; **MAKAROV V et al.** Tumor and Microenvironment Evolution during Immunotherapy with Nivolumab.. *Cell*, 2017, vol. 171 (4), 934-949. e16
- **STEFF A-M** ; **FORTIN M** ; **ARGUIN C** ; **HUGO P**. Detection of a decrease in green fluorescent protein fluorescence for the monitoring of cell death: An assay amenable to high throughput screening technologies.. *Cytometry.*, 2001, vol. 45 (4), 237-243
- **BOSSELUT R** ; **HUSEBY E** ; **OAKES T et al.** Quantitative Characterization of the t Cell Receptor Repertoire of Naive and Memory subsets Using an Integrated experimental and Computational Pipeline Which Is Robust, economical, and Versatile.. *Immunol.*, 2017, vol. 8, 1267
- **GREIFF V** ; **MIHO E** ; **MENZEL U** ; **REDDY ST**. Bioinformatic and Statistical Analysis of Adaptive Immune Repertoires.. *Trends Immunol.*, 2015, vol. 36 (11), 738-749

- **EMERSON RO** ; **DEWITT WS** ; **VIGNALI M et al.** Immunosequencing identifies signatures of cytomegalovirus exposure history and HLA-mediated effects on the T cell repertoire.. *Nat Genet.*, 2017, vol. 49 (5), 659-665
- **SETHNA Z** ; **ELHANATI Y** ; **CALLAN CG** ; **WALCZAK AM** ; **MORA T**. OLGA: fast computation of generation probabilities of B- and T-cell receptor amino acid sequences and motifs.. *Bioinformatics.*, 2019, vol. 35 (17), 2974-2981
- **DEWITT WS** ; **SMITH A** ; **SCHOCH G** ; **HANSEN JA** ; **MATSEN FA** ; **BRADLEY P**. Human T cell receptor occurrence patterns encode immune history, genetic background, and receptor specificity.. *Elife.*, 2018, 7
- **ZHAO Y** ; **ZHENG Z** ; **COHEN CJ et al.** High-efficiency transfection of primary human and mouse T lymphocytes using RNA electroporation.. *Mol Ther.*, 2006, vol. 13 (1), 151-159
- **COHEN CJ** ; **ZHAO Y** ; **ZHENG Z** ; **ROSENBERG SA** ; **MORGAN RA**. Enhanced antitumor activity of murine-human hybrid T-cell receptor (TCR) in human lymphocytes is associated with improved pairing and TCR/CD3 stability.. *Cancer Res.*, 2006, vol. 66 (17), 8878-8886
- **HE L** ; **HAKIMI J** ; **SALHA D** ; **MIRON I** ; **DUNN P** ; **RADVANYI L**. A sensitive flow cytometrybased cytotoxic T-lymphocyte assay through detection of cleaved caspase 3 in target cells.. *J Immunol Methods.*, 2005, vol. 304 (1-2), 43-59